# EUROPEAN PATENT SPECIFICATION

(11) **EP 0 648 280 B1**
(45) Date of publication and mention of the grant of the patent: **12.05.1999**
(21) Application number: 91903942.0
(22) Date of filing: 15.02.1991
(51) Int. Cl.: C12Q 1/68

(54) **METHOD AND REAGENT FOR DETERMINING SPECIFIC NUCLEOTIDE VARIATIONS**
VERFAHREN UND REAGENS ZUM NACHWEIS VON SPEZIFISCHEN NUKLEOTID-VARIATIONEN
PROCEDE ET REACTIF PERMETTANT DE DETERMINER DES VARIATIONS SPECIFIQUE DE NUCLEOTIDES

(30) Priority: 16.02.1990 US 482005
(43) Date of publication of application: 19.04.1995
(73) Proprietor: SANGTEC MEDICAL AB, 161 02 Bromma (SE)
(72) Inventor: SÖDERLUND, Hans, SF-01940 Espoo (FI); SYVÄNEN, Ann-Christine, SF-00270 Helsinki (FI)
(74) Representative: Fagerlin, Heléne
(86) International application number: FI9100046
(87) International publication number: WO9113075

(56) References cited:
- EP-A- 0 123 513
- EP-A- 0 317 074
- EP-A- 0 332 435
- EP-A- 0 333 465
- EP-A- 0 357 011
- EP-A- 0 370 694
- EP-A- 0 371 437
- WO-A-89/09835
- WO-A-89/10414
- WO-A-90/09455
- WO-A-90/11372
- GB-A- 2 202 328
- US-A- 4 851 331

### SEQUENCE LISTING

### (1) GENERAL INFORMATION:

(i) APPLICANT: Orion-yhtymä Oy
(ii) TITLE OF INVENTION: Method and Reagent for Determining Specific Nucleotide Variations
(iii) NUMBER OF SEQUENCES: 27
(iv) CORRESPONDENCE ADDRESS:
   Orion Corporation
   ORION PHARMACEUTICA,
   Patent Depatment
   P.O.Box 65
   02102 ESPOO
   FINLAND
(v) COMPUTER READABLE FORM: <>
   (A) MEDIUM TYPE: <>
   (B) COMPUTER: <>
   (C) OPERATING SYSTEM: <>
   (D) SOFTWARE: <>
(vi) CURRENT APPLICATION DATA:
   (A) APPLICATION NUMBER: To be determined
   (B) FILING DATE:
   (C) CLASSISFICATION:

### (2) INFORMATION FOR SEQ ID NO:1:

**(i) SEQUENCE CHARACTERISTICS:**
   **(A) LENGHT:** 22 bases
   **(B) TYPE:** nucleic acid
   **(C) TOPOLOGY:** linear
**(ii) MOLECULE TYPE:** DNA
**(xi) SEQUENCE DESCRIPTION: SEQ ID NO:1:**

AAG GAG TTG AAG GCC TAC AAA T   22

### (2) INFORMATION FOR SEQ ID NO:2:

**(i) SEQUENCE CHARACTERISTICS:**
   **(A) LENGHT:** 22 bases
   **(B) TYPE:** nucleic acid
   **(C) TOPOLOGY:** linear
**(ii) MOLECULE TYPE:** DNA
**(xi) SEQUENCE DESCRIPTION: SEQ ID NO:2:**

GAA CAA CTG AGC CCG GTG GCG G 22

### (2) INFORMATION FOR SEQ ID NO:3:

**(i) SEQUENCE CHARACTERISTICS:**
   **(A) LENGHT:** 20
   **(B) TYPE:** nucleic acid
   **(C) TOPOLOGY:** linear
**(ii) MOLECULE TYPE:** DNA
**(xi) SEQUENCE DESCRIPTION: SEQ ID NO:3:**

GCG CGG ACA TGG AGG ACG TG  20

### (2) INFORMATION FOR SEQ ID NO:4:

**(i) SEQUENCE CHARACTERISTICS:**
   **(A) LENGHT:** 20
   **(B) TYPE:** nucleic acid
   **(C) TOPOLOGY:** linear
**(ii) MOLECULE TYPE:** DNA
**(xi) SEQUENCE DESCRIPTION: SEQ ID NO:4:**

ATG CCG ATG ACC TGC AGA AG 20

### (2) INFORMATION FOR SEQ ID NO:5:

**(i) SEQUENCE CHARACTERISTICS:**
   **(A) LENGHT:** 22
   **(B) TYPE:** nucleic acid
   **(C) TOPOLOGY:** linear
**(ii) MOLECULE TYPE:** DNA
**(xi) SEQUENCE DESCRIPTION: SEQ ID NO:5:**

TCG CGG GCC CCG GCC TGG TAC A 22

### (2) INFORMATION FOR SEQ ID NO:6:

**(i) SEQUENCE CHARACTERISTICS:**
   **(A) LENGHT:** 22
   **(B) TYPE:** nucleic acid
   **(C) TOPOLOGY:** linear
**(ii) MOLECULE TYPE:** DNA
**(xi) SEQUENCE DESCRIPTION: SEQ ID NO:6**

GGA TGG CGC TGA GGC CGC GCT C   22

### (2) INFORMATION FOR SEQ ID NO:7:

**(i) SEQUENCE CHARACTERISTICS:**
   **(A) LENGHT:** 20
   **(B) TYPE:** nucleic acid
   **(C) TOPOLOGY:** linear
**(ii) MOLECULE TYPE: DNA**
**(xi) SEQUENCE DESCRIPTION: SEQ ID NO:7:**

GTA CTG CAC CAG GCG GCC GC 20

### (2) INFORMATION FOR SEQ ID NO:8:

**(i) SEQUENCE CHARACTERISTICS:**
   **(A) LENGHT:** 22
   **(B) TYPE:** nucleic acid
   **(C) TOPOLOGY:** linear
**(ii) MOLECULE TYPE:** DNA
**(xi) SEQUENCE DESCRIPTION: SEQ ID NO:8:**

GGC CTG GTA CAC TGC CAG GC 22

### (2) INFORMATION FOR SEQ ID NO:9:

**(i) SEQUENCE CHARACTERISTICS:**
   **(A) LENGHT:** 20
   **(B) TYPE:** nucleic acid
   **(C) TOPOLOGY:** linear
**(ii) MOLECULE TYPE:** DNA
**(xi) SEQUENCE DESCRIPTION: SEQ ID NO:9:**

CAT TTG CTT CTG ACA CAA CT  20

### (2) INFORMATION FOR SEQ ID NO:10:

**(i) SEQUENCE CHARACTERISTICS:**
   **(A) LENGHT:** 20
   **(B) TYPE:** nucleic acid
   **(C) TOPOLOGY:** linear
**(ii) MOLECULE TYPE:** DNA
**(xi) SEQUENCE DESCRIPTION: SEQ ID NO:10:**

CAT GGT GCA CCT GAC TCC TG 20

### (2) INFORMATION FOR SEQ ID NO:11:

**(i) SEQUENCE CHARACTERISTICS:**
   **(A) LENGHT:** 20
   **(B) TYPE:** nucleic acid
   **(C) TOPOLOGY:** linear
**(ii) MOLECULE TYPE:** DNA
**(xi) SEQUENCE DESCRIPTION: SEQ ID NO:11:**

CAA CTT CAT CCA CGT TCA CC  20

### (2) INFORMATION FOR SEQ ID NO:12:

**(i) SEQUENCE CHARACTERISTICS:**
   **(A) LENGHT:** 20
   **(B) TYPE:** nucleic acid
   **(C) TOPOLOGY:** linear
**(ii) MOLECULE TYPE: DNA**
**(xi) SEQUENCE DESCRIPTION: SEQ ID NO:12:**

CAG TAA CGG CAG GCG GCC GC  20

### (2) INFORMATION FOR SEQ ID NO:13:

**(i) SEQUENCE CHARACTERISTICS:**
   **(A) LENGHT:** 23
   **(B) TYPE:** nucleic acid
   **(C) TOPOLOGY:** linear
**(ii) MOLECULE TYPE:** DNA
**(xi) SEQUENCE DESCRIPTION: SEQ ID NO:13:**

CTG GAG CCT TCA GAG GGT AAA AT   23

### (2) INFORMATION FOR SEQ ID NO:14:

**(i) SEQUENCE CHARACTERISTICS:**
   **(A) LENGHT:** 24
   **(B) TYPE:** nucleic acid
   **(C) TOPOLOGY:** linear
**(ii) MOLECULE TYPE:** DNA
**(xi) SEQUENCE DESCRIPTION: SEQ ID NO:14:**

TGG CAC CAT TAA AGA AAA TAT CAT 24

### (2) INFORMATION FOR SEQ ID NO:15:

**(i) SEQUENCE CHARACTERISTICS:**
   **(A) LENGHT:** 23
   **(B) TYPE:** nucleic acid
   **(C) TOPOLOGY:** linear
**(ii) MOLECULE TYPE:** DNA
**(xi) SEQUENCE DESCRIPTION: SEQ ID NO:15:**

CAT GCT TTA ATG ACG CTT CTA TA 23

### (2) INFORMATION FOR SEQ ID NO:16:

**(i) SEQUENCE CHARACTERISTICS:**
   **(A) LENGHT:** 21
   **(B) TYPE:** nucleic acid
   **(C) TOPOLOGY:** linear
**(ii) MOLECULE TYPE:** DNA
**(xi) SEQUENCE DESCRIPTION: SEQ ID NO:16:**

ATG ACT GAA TAT AAA CTT GTG 21

### (2) INFORMATION FOR SEQ ID NO:17:

**(i) SEQUENCE CHARACTERISTICS:**
   **(A) LENGHT:** 21
   **(B) TYPE:** nucleic acid
   **(C) TOPOLOGY:** linear
**(ii) MOLECULE TYPE:** DNA
**(xi) SEQUENCE DESCRIPTION: SEQ ID NO:17**:

TTC GTC CAC AAA ATG ATT CTG 21

### (2) INFORMATION FOR SEQ ID NO:18:

**(i) SEQUENCE CHARACTERISTICS:**
   **(A) LENGHT:** 21
   **(B) TYPE:** nucleic acid
   **(C) TOPOLOGY:** linear
**(ii) MOLECULE TYPE:** DNA
**(xi) SEQUENCE DESCRIPTION: SEQ ID NO:18:**

AAG GCA CTC TTG CCT ACG CCA   21

### (2) INFORMATION FOR SEQ ID NO:19:

**(i) SEQUENCE CHARACTERISTICS:**
   **(A) LENGHT:** 21
   **(B) TYPE:** nucleic acid
   **(C) TOPOLOGY:** linear
**(ii) MOLECULE TYPE:** DNA
**(xi) SEQUENCE DESCRIPTION: SEQ ID NO:19:**

AGG CAC TCT TGC CTA CGC CAC   21

### (2) INFORMATION FOR SEQ ID NO:20:

**(i) SEQUENCE CHARACTERISTICS:**
   **(A) LENGHT:** 18
   **(B) TYPE:** nucleic acid
   **(C) TOPOLOGY:** linear
**(ii) MOLECULE TYPE:** DNA
**(xi) SEQUENCE DESCRIPTION: SEQ ID NO:20:**

AAC TTG TGG TAG TTG GAG CT 18

### (2) INFORMATION FOR SEQ ID NO:21:

**(i) SEQUENCE CHARACTERISTICS:**
   **(A) LENGHT:** 18
   **(B) TYPE:** nucleic acid
   **(C) TOPOLOGY:** linear
**(ii) MOLECULE TYPE:** DNA
**(xi) SEQUENCE DESCRIPTION: SEQ ID NO:21:**

ACT TGT GGT AGT TGG AGC TG 18

### (2) INFORMATION FOR SEQ ID NO:22:

**(i) SEQUENCE CHARACTERISTICS:**
   **(A) LENGHT:** 20
   **(B) TYPE:** nucleic acid
   **(C) TOPOLOGY:** linear
**(ii) MOLECULE TYPE:** DNA
**(xi) SEQUENCE DESCRIPTION: SEQ ID NO:22:**

GAC TGA GTA CAA ACT GGT GG 20

### (2) INFORMATION FOR SEQ ID NO:23:

**(i) SEQUENCE CHARACTERISTICS:**
   **(A) LENGHT:** 20
   **(B) TYPE:** nucleic acid
   **(C) TOPOLOGY:** linear
**(ii) MOLECULE TYPE:** DNA
**(xi) SEQUENCE DESCRIPTION: SEQ ID NO:23:**

CTC TAT GGT GGG ATC ATA TT 20

### (2) INFORMATION FOR SEQ ID NO:24:

**(i) SEQUENCE CHARACTERISTICS:**
   **(A) LENGHT:** 20
   **(B) TYPE:** nucleic acid
   **(C) TOPOLOGY:** linear
**(ii) MOLECULE TYPE:** DNA
**(xi) SEQUENCE DESCRIPTION: SEQ ID NO:24:**

ACT GGT GGT GGT TGG AGC AG 20

### (2) INFORMATION FOR SEQ ID NO:25:

**(i) SEQUENCE CHARACTERISTICS:**
   **(A) LENGHT:** 21
   **(B) TYPE:** nucleic acid
   **(C) TOPOLOGY:** linear
**(ii) MOLECULE TYPE:** DNA
**(xi) SEQUENCE DESCRIPTION: SEQ ID NO:25:**

GAG AAT CCA TAC AAT ACT CCA 21

### (2) INFORMATION FOR SEQ ID NO:26:

**(i) SEQUENCE CHARACTERISTICS:**
   **(A) LENGHT:** 21
   **(B) TYPE:** nucleic acid
   **(C) TOPOLOGY:** linear
**(ii) MOLECULE TYPE:** DNA
**(xi) SEQUENCE DESCRIPTION: SEQ ID NO:26:**

TAA CCC ATC CAA AGG AAT GGA 21

### (2) INFORMATION FOR SEQ ID NO:27:

**(i) SEQUENCE CHARACTERISTICS:**
   **(A) LENGHT:** 20
   **(B) TYPE:** nucleic acid
   **(C) TOPOLOGY:** linear
**(ii) MOLECULE TYPE:** DNA
**(xi) SEQUENCE DESCRIPTION: SEQ ID NO:27:**

ATC TGT TGA GGT GGG GAC TT   20

## Description

### TECHNICAL FIELD

The present invention relates to a method and reagents for determining specific nucleotide variations in a defined polynucleotide region, and to the use of this method in identifying specific point mutations and genetic variations.

### BACKGROUND OF THE INVENTION

The genetic information of living organisms is carried in the nucleotide sequence of their genome. In the process of gene expression the nucleotide sequence is translated to amino acid sequences, i.e. proteins. Minor changes in the nucleotide sequence, even a single base substitution, may result in an altered protein product. The altered quality or quantity of given proteins changes the phenotype (i.e. the observable characteristics) of the organism or the cell, which for instance may be observed as a development of a disease.

The knowledge of the exact molecular defects causing inherited diseases, as well as predisposition to genetic disorders and cancer is increasing rapidly. The knowledge of the relevance of somatic mutations in malignancies is, however, limited due to the lack of rapid and reliable assay procedures for screening large numbers of samples.

Inherited diseases caused by point mutations include sickle cell anemia and β-thalassemias, which are caused by mutations in the β-globin gene. (Antonarakis, 1989, New England J Med, Vol. 320, pp. 153-163) These mutations generally involve the replacement, insertion or deletion of one to four nucleotides from the sequence of the normal gene. Sickle cell anemia is caused by homozygosity for one unique base pair substitution in the sixth codon of the β-globin gene. (Antonarakis, supra) A large number of mutations in the β-globin gene that can lead to β-thalassemia have been characterized (Antonarakis, supra).

Other known inherited diseases caused by point mutations include α-thalassemia, phenylketonuria, hemophilia, α₁-anti-trypsin deficiency (Antonarakis, supra) and cystic fibrosis.

Cystic fibrosis is the most common autosomal recessive genetic disorder. It affects about 1/2000 individuals of Caucasian populations and consequently the carrier frequecy is about 5%. The recent cloning and genetic analysis of the cystic fibrosis transmembrane regulator (CFTR) gene (Kerem et al., 1989, Science, Vol 245, pp. 1073-1080) has revealed one major mutation, denoted ΔF508, which is a deletion of three nucleotides leading to loss of the phenylalanine at amino acid residue 508. The prevalence of this mutation is on the average 68% in North American and European patient populations, the range being 40 - 88% in reports containing more than 100 CF chromosomes. Because of the high frequency of cystic fibrosis, efficient methods for the screening of carriers and for prenatal diagnosis are needed in the risk group countries.

An example of a polymorhism, which correlates to predisposition to disease is the three-allelic polymorphism of the apolipoprotein E gene. This polymorphism is due to single base substitutions at two DNA loci on the Apo E gene (Mahley, 1988, Science, Vol. 240, pp. 622-630). It may explain as much as 10 % of the individual variations in the serum cholesterol levels. More than 90 % of patients with type III hyperlipoproteinemia are homozygous for one of the Apo E alleles.

The human major histocompatibilty complex is a polymorphic system of linked genes located within a conserved region of the genome. The class II genes within the HLA-D (human leukocyte antigen) region encode a series of highly polymorphic alleles (Thomson, 1988, Annu. Rev. Genet., 22:31-50; Morel et al., 1988, Proc. Natl. Acad. Sci. USA, 85:8111-8115; Scharf et al., 1988, Proc. Natl. Acad. Sci. USA, 85:3504-3508). This polymorphism has been shown to be associated with susceptibility to autoimmune diseases, such as insulin-dependent diabetes and pemphigus vulgaris.

The human ras-gene family, which includes the homologous H-, K- and N-ras genes, is one of the potential targets for mutational changes that play a role in human tumorigenesis. Point mutations in either codon 12, 13 or 61 of the ras genes have been shown to convert these genes into transforming oncogenes (Bos et al., 1985, Nature, Vol. 315, pp. 726-730). Somatic point mutations in the N-ras gene have been detected in association with acute myeloid leukemias (AML) (Farr et al., 1988, Proc. Natl. Acad. Sci. USA, 85:1629-1633) and other hemotological malignancies (Neri et al., 1988, Proc. Natl. Acad. Sci. USA, 85:9268-9272). A method for sensitive detection of the N-ras mutations in small quantities of leukemic cells amongst a wast majority of normal cells would consitute a most valuable tool in the follow-up of therapy of AML and other N-ras associated malignancies.

The detection of the specific base changes in the first an second position of codons 12, 13 and 61 of the N-ras gene requires either hybridization with a large number of different oligonucleotide probes, or direct nucleotide sequence determination of the amplified DNA. One critical point in both approaches is the proportion of cells containing the mutation. Depending on the method of choice a mutation must be present in 5-20% of the analyzed cell population to be detectable.

Point mutations and genetic variations in microorganisms might lead to altered pathogenicity or resistancy to therapeutics. The human immunodeficiency virus (HIV-1) can develop mutants which are resistant to zidovudine (AZT). The resistant virus isolates contain several point mutations, but three mutations seem to be common to all resistant strains: Asp 67 - Asn (GAC - AAC), Lys 70 - Arg (AAT - GAT)and Thr 215 - Phe ( ACC - TTC)or Tyr (ACC - TAC) ( Larder and Kemp, 1989, Science 246:1155-1158)

It would therefore be significant if changes in nucleotide sequences in the genome of living organisms could be determined accurately and with such efficiency and ease that large numbers of samples could be screened. This would afford opportunities for pre- or postnatal diagnosis of hereditary predispositions or diseases and for detection of somatic mutations in cancer. Such a method could also be used for the selection of cells and strains for industrial biotechnology and for plant and animal breading. Presently available methods suffer from drawbacks limiting their routine use.

Polymorphisms or mutations in DNA sequences are most commonly detected by hybridization to allele-specific oligonucleotide (ASO) probes. The nucleotide sequence of the ASO probes is designed to form either a perfectely matched hybrid or to contain a mismatched base pair at the site of the variable nucleotide residues. The distinction between a matched and a mismatched hybrid is based on i) differences in the thermal stability of the hybrids in the conditions used during hybridization or washing (European Patent Publication EP-237362), ii) differences in the stability of the hybrids analyzed by denaturing gradient electrophoresis or iii) chemical cleavage at the site of the mismatch (European Patent Publication EP-329311).

Oligonucleotides with 3'ends complementary to the site of the variable nucleotides have been used as allele-specific primers (European Patent Publication EP-332435). The identification of the variable nucleotide is based on the fact that a mismatch at the 3'end inhibits the polymerization reaction. A similar approach is used in oligomer ligation assays, in which two adjacent oligonucleotides are ligated only if there is a perfect match at the termini of the oligonucleotides (European Patent Publication EP-336731).

Cleavage of the DNA sequence with restriction enzymes can be utilized for identification of the variation, provided that the variable nucleotide alters, e.g. creates or destroys, a specific restriction site. Nucleotide sequencing is the most informative method for the determination of variable nucleotides.

The methods referred to above are relatively complex procedures, suffering from drawbacks making them difficult to use in routine diagnostics. The use of allele specific oligonucleotide probes requires careful optimization of the rection conditions separately for each application. Fractionation by gel electrophoresis is required in several of the methods above. Such methods are not easily automatized.

WO 89/10414 describes a process for the detection of mutations based on primer extension which is preceded by nucleic acid amplification and immobilisation of target sequences. The PCR reaction is performed in solution with both strands and rehybridisation beetween the two strands of the PCR product compete with the hybridisation reaction of the detection primer.

EP 0 123 513 B1 relates to a method for detecting mutation of a specific nucleotide base by extension with a complementary probe and polymerase enzyme digestion.

In the applicant's own GB 2 202 328 a method is described for assaying nucleic acids by hybridisation using capturing probes acting as modified primers incorporated into copies of the target nucleic acid produced by polymerisation.

### SUMMARY OF THE INVENTION

We have now developed an improved method, which allows the detection of nucleotide variations. This method provides several advantages over prior art methods. The method according to this invention comprises few and easily performed procedures. It is especially suited for routine determinations of point mutations and nucleotide variations, such as single mis-matches, deletions, insertions and inversions. The method according to the present invention allows quantification of the proportion of mutated cells in a sample as well as identification of mutations present in as little as 0.5% of the analyzed cell population. Furthermore the complete protocol of the method disclosed is easily automated, which is becoming increasingly important in routine diagnostics.

The method of detection of the variable nucleotide(s) is based on primer extension and incorporation of detectable nucleoside triphosphates in the detection step. By selecting the detection step primers from the region immediately adjacent to the variable nucleotide, this variation can be detected after incorporation of as few as one nucleoside triphosphate.

Labelled nucleoside triphosphates matching the variable nucleotide are added and the incorporation of a label into the detection step primer is measured.

The selection of the detection step primers is important to the method according to this invention and is dependent on the nucleotide sequence of interest. The detection step primers are preferably selected so as to span the region immediately toward the 3' end from the variable nucleotide to be detected. The detection step primers can also be complemetary to a sequence beginning several nucleotides removed from the variable nucleotide. The only limitation concerning the position of the detection step primers is that the sequence between the 3' end of the detection step primer and the variable nucleotide to be detected must not contain a nucleotide residue of the same type as the one to be detected. The detection step primers can equally well be chosen to be complementary to either the coding or non-coding strands of the nucleotide sequence of interest.

The target nucleic acid is enriched by amplification in vitro to allow detection of one single variable nucleotide in the human genome. The present invention advantageously employs an earlier disclosed method for amplification and affinity modification of the target nucleic acid to be detected. In the present method the target nucleic acid containing the variable nucleotide(s) is immobilized on a solid support permitting the removal of the amplification mixture from the target nucleic acid.

The present invention also encompasses individual primer reagents and reagent combinations or kits for practising the method of the invention. While the precise reagents and packaging will depend on the type of nucleotide variations or point mutations to be detected, such a kit will in general include at least one modified amplification primer having an attachment moiety included and at least one detections step primer, but may also include at least one support adapted to immobilize the modified copies of the target nucleic acid and at least one labelled nucleoside triphosphate.

Methods and kits of the present invention are defined in the appended claims.

### BRIEF DESCRIPTION OF THE DRAWINGS

Figure 1 illustrates a set-up for a test according to the disclosed method in the case where one variable nucleotide residue (X₁ or X₂) is detected.

Figure 2 illustrates a set-up for a test according to the disclosed method in which case the detection step primer is chosen to span the nucleotide sequence n nuclotides from the variable nucleotide to be detected.

Figure 3 illustrates a set-up for a test according to the disclosed method, in a case where a plurality of mismatches adjacent to each other, is to be detected. In this case the test is performed in two steps, whereby the detection step primer 1 is eluted before step two is performed.

### Symbols used in the figures:

- X and X' denote nucleotide residues
- The variable nucleotide to be detected is denoted either X₁, X₂, X₃ or X₄
- Y₁, Y₂, Y₃ and Y₄ denote nucleotides complementary to X₁ , X₂ , X₃ or X₄, respectively
- dY denotes a deoxyribonucloside triphosphate
- dY* and dY⁺ denote labelled deoxyribonucleoside triphophates
- ddY denotes a dideoxyribonucleoside triphosphate
- ddY* and ddY⁺ denote labelled dideoxyribonucleoside triphophates
- * and + denote different detectable labels

### DETAILED DESCRIPTION OF THE INVENTION

The present invention resides in a method to determine a specific nucleotide variation in a previously defined region of a nucleic acid present in a complex nucleic acid mixture. This defined region containing the nucleotide variation is herein referred to as the "target nucleid acid".

The method according to the invention is based on a primer extension reaction, using at least one detection step primer complementary to the nucleotide sequence 3' from the variable nucleotide to be detected. The detection of the variable nucleotide is performed by detecting a labelled nucleoside triphospate incorporated by extension of the detection step primer.

The target nucleic acid is amplified in vitro prior to the detection step to provide a detectable amount of the target nucleic acid. The actual quantity of nucleic acid will vary depending on the labeling moiety employed in the detection step and the sensitivity of analytical techniques for that moiety. According to the present invention, an affinity moiety is introduced into copies of the region of interest of the target nucleic acid. This is conveniently done by a primer dependent amplification reaction using an affinity labelled amplification primer. The copies of the target nucleic acid molecules are immobilized on a solid support with the aid of the introduced affinity moiety.

Different set-ups of the test are described below in the description of the preferred embodiments and further illustrated by the examples. It will be evident to one skilled in the art, that the possibilities of constructing the test are not limited to these given examples and that the set-up of the test is determined by the given mutation or nucleotide variation to be detected.

### a) Introduction of affinity moieties into copies of the target DNA

The source of the target nucleic acid (DNA or RNA) suspected to contain the variable nucleotide residue can be any human, animal or plant cell or a microbe. The target nucleic acid can be isolated from biological samples by conventional nucleic acid purification methods, but according to the present invention it is possible to use crude biological samples.

The target nucleic acid comprising the variable nucleotide site of interest is multiplied in vitro using one or more primers close to the nucleotide sequence of interest. The term "amplification" as used herein refers to any primer dependent elongation of a nucleotide sequence with the aid of a polymerizing agent. Suitable primer dependent elongation reactions are for example the polymerase chain reaction (Kleppe et al., 1971, J. Mol. Biol. 56:341-361; and U.S. Patent 4,683,202), processes utilizing both primer dependent replication and independent transcription (European Patent Publication EP-329822) or ligation amplification reactions (PCT Patent Publication WO-89/09835).

We have previously developed a convenient method of nucleotide sequencing, which is well adapted for use as a routine diagnostic tool (EP-A-0 371 437). This method makes use of the affinity based hybrid collection method disclosed in GB-A-2 202 328. The target DNA to be tested is amplified in vitro prior to sequencing. In this method one of the primers used in the amplification reaction comprises an attachment moiety such as biotin. The enriched fragment is captured on a solid matrix, to which streptavidin or avidin has been attached. The excess reagents are completely removed by washing the matrix. The captured fragment is rendered single stranded and the chain termination reactions are carried out directly on the matrix. The products of the chain termination reaction are released and the nucleotide sequence is determined after polyacryamide gel electrophoresis. This diagnostically suitable solid phase sequencing method provides several advantages over the prior art methods for determining nucleotide variations described above, but still contains the laborious, expensive and skill-requiring sequencing gel electrophoresis step.

According to the present invention the amplification of the target nucleic acid comprising the suspected nucleotide variation is conveniently done by using a modified primer introducing affinity moieties into the copies of the target nucleic acid sequence. In such an amplification one of the amplification primers are modified to include attachment moieties. By selecting which amplification primer is modified, it is possible to determine which strand of a double stranded DNA the variable site is detected on. The target sequence of interest is amplified with a suitable number of cycles of this modified polymerase chain reaction.

As a result of this process, copies of the original target nucleic acid sequence, now modified with attachment moieties, are obtained by incorporation of the modified primers into the synthesized polynucleotide molecules.

The term "attachment moiety" as used herein refers to any component having affinity for another component which forms an affinity pair with that other component. For example, biotin - avidin/streptavidin, antigens or haptens - antibodies, heavy metal derivatives - thiogroups, various polynucleotides such as homopolynucleotides as poly dG - poly dC, poly dA- poly dT and poly dA- poly U, are such affinity pairs. Any component pairs with strong affinity for each other can be used as the affinity pair. Suitable affinity pairs are also found among ligands and conjugates used in immunological methods. An "attachment moiety" is an affinity moiety or a moiety providing a site for the attachment of an affinity moiety.

The term "modified amplification primer" as used herein refers to an oligonucleotide primer modified as to contain an attachment moiety. The oligonucleotide primers may be synthesized by standard chemical methods or prepared by recombinant DNA techniques. The essential feature of the amplification primer is that it is able to specifically bind by base pairing to the original target nucleotide sequence of interest at a point in the sequence such that the site of interest will be amplified. Thus the amplification must be complementary to a region of the target nucleotide sequence between the 3' end of the target nucleic acid and the site of interest. The 3' end of the amplification primer is preferably complementary to the target nucleic acid sequence at a point less than 100 residues removed from the site of interest due to liminations in polymerase enzyme processivity. The size of the primers is preferably between 14 and 40 bases, but primers as short as 8 bases or considerably longer than 40 based may be used. The primers are modified with the attachment moieties using chemical or enzymatic or any other method. Preferably the attachment moiety is attached to the 5' end of the primer. Other sites of attachment are also possible, provided that the base pairing property of the primer and its ability to function in an elongation reaction are retained.

### b) Separation of the target nucleic acid copies.

After amplification, the target nucleic acid copies are separated from the amplification mixture. Copies of the target nucleic acid molecules containing the attachment moieties are immobilized on a solid matrix with the aid of a complementary attachment site, e.g. the other component of the affinity pair. The matrix is then washed to remove all unbound material, such as nucleotide triphosphates and primers, that could interfere with the reactions during the detection step. The immobilized target nucleic acid is rendered single-stranded either before or after the immobilization step. The immobilization of the modified target nucleic acid copies makes it possible to reuse the target sequence if multiple determinations are to be performed on the same target sequence of interest.

The term "solid matrix" as used herein refers to any format, such as beads, microparticles, the surface of a microtitration well or a test tube, a dipstick or a filter. The material of the matrix may be polystyrene, cellulose, latex, nitrocellulose, nylon, polyacrylamide, dextran or agarose. The only prerequisite for the material is that the attachment site can be attached to it.

### c) The detection step primer

After separation from the amplification mixture, the single-stranded DNA fragment is allowed to hybridize to a primer, the detection step primer, which is complementary to the nucleotide sequence 3' of the variable nucleotide. This can be carried out in an immobilized state or in solution.

The term "detection step primer" as used herein refers to an oligo- or polynucleotide primer, which functions as the point of initiation for the primer dependent elongation. The detection step primer is selected as to be hybridizable to a nucleotide sequence immediately or closely adjacent to the variable nucleotide to be detected. The detection step primer can be chosen as to be complementary to either the coding or the non-coding strand of a double stranded target, depending on which strand was immobilized in step b) described above. The detection step primer can be modified, e.g. with an affinity moiety as described in section a) above but using a different affinity moiety. Preferably the detection step primer is not modified.

The selection of the detection step primers is determined by the nature of the nucleotide variation to be detected.

In a preferred embodiment of the method according to the present invention, the detection step primer is selected as to be immediately adjacent to the variable nucleotide to be detected.

In another embodiment of the method according to the present invention the detection step primer is selected to be hybridizable to a nucleotide sequence n nucleotide residues away from the variable nucleotide to be detected. The only limitation as to the number n of nucleotide residues between the 3' end of the detection step primer and the variable nucleotide is that there must be no nucleotide residues identical to the one(s) to be detected among these n nucleotide residues.

In another embodiment of the method according to the present invention two or more variable nucleotide residue are identified. In this case it is necessary to design at least two different detection step primers.

The set-up of the test depending on the nucleotide variations to be detected are further described below.

### d) The detection step primer extension

The detection step primer is annealed to the copies of the target nucleic acid and a solution containing one or more nucleoside triphosphates including at least one labelled or modified nucleoside triphosphate, is added together with a polymerizing agent in conditions favoring primer extension. Either labelled deoxyribonucleoside triphosphates (dNTPs) or chain terminating dideoxyribonucleoside triphosphates (ddNTPs) can be used. The polymerizing agent will extend the primer with the nucleoside triphoshate complementary to the variable nucleotide adjacent to the primer. The extended nucleic acid may be immobilized during this detection step primer extension, for example, via affinity-binding of a modified amplified copy, or it may be immobilized afterwards, for example, via an affinity-modified detector primer. In either case, after washing the affinity-matrix, the incorporated label is measured directly on the matrix or after elution.

The term "labelled nucleoside triphosphate" as used herein refers to any nucleoside triphosphate, deoxy- or dideoxynucleosidetriphosphte, labelled with a detectable label or modified as to comprise an attachment moiety capable of binding a detectable label. The method according to the invention is not dependent on the label used, although sensitivity will vary depending on the detectability of the label. The only limitation as to the choice of the detectable label is that it will not disturb the incorporation of the labelled nucleoside triphosphate during the polymerization reaction.

The term "polymerizing agent" as used herein refers to any enzyme which is capable of primer dependent elongation of nucleic acids. Suitable enzymes include, for example, T7 DNA polymerase, T4 DNA polymerase, the Klenow fragment of Escherichia coli DNA polymerase and other suitable DNA polymerases, reverse transcriptase and polymerases from thermophilic microbes such as Thermus aquaticus and Thermus thermophilus.

### e) Preferred modes of detecting nucleotide variations

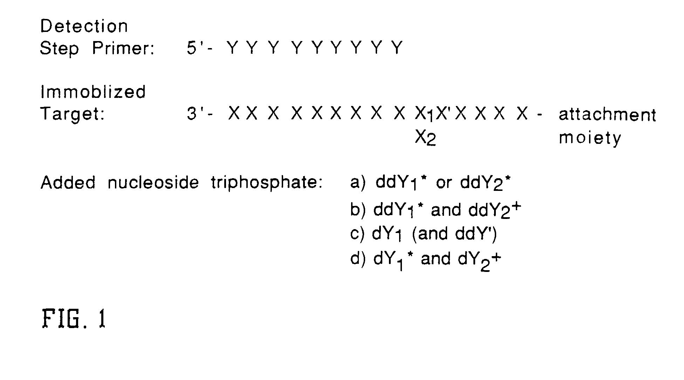

Figure 1 illustrates schematically an embodiment of the invention. The target nucleic acid is represented by X's, with X₁ and X₂ representing two alternative nucleotide residues at the site to be investigated. The detection step primer illustrated in Fig. 1 is represented by Y's, and is complementary to the portion of the target sequence starting immediately 3'-ward of the site to be investigated. In practising the invention the detection step primer is hybridized to the target sequence, and a selected nucleoside triphosphate or a mixture of nucleoside triphosphates is added and a chain extension reaction is allowed to proceed under conditions favorable for elongation of the primer.

In the simplest embodiment of the invention, the nucleoside triphosphate mixture contains just the labelled dideoxynucleoside triphosphate (ddNTP) corresponding to either X₁ or X₂ (option a in Fig 1). The incorporation of a ddNTP terminates the primer extension reaction after the incorporation of only this one nucleotide, whereafter it is possible to determine the variable nucleotide by detecting the incorporated label of the added nucleotide.

This embodiment is especially suitable when the nucleotide variation to be detected consists of one single point mutation (X₁ → X₂). The sample can be divided into two parts, whereafter X₁ is detected from one of the samples and X₂ from the other. This allows the identification of heterozygous samples. It is equally possible to add two or more different and differently labelled ddNTPs to an undivided sample (option b in Fig 1). In this embodiment it is possible to determine more than one point mutation occuring at the same site out of one undivided sample (X₁ → X₂ , X₃ or X₄).

It is also possible to use a labelled deoxynucleoside triphosphate (dNTP), which corresponds to the variable nucleotide to be detected and to determine the incorporation of this label. When a labelled dNTP is used, it is advantageous, but not necessary, to add unlabelled ddNTPs corresponding to the other three nucleotide residues (option c in Fig 1). The addition of chain terminating ddNTPs provides a means for preventing incorporation of possibly remaining NTPs from the modification step (a).

Yet another possibility is to use two or more different, differently labelled dNTPs making it possible to detect heterozygotes in an undivided sample. When using more than one labelled dNTP the results may be difficult to interpret if the nucleotide residue x following the variable nucleotide residue in the target sequence is identical to either of the added ones (option d in Fig 1).

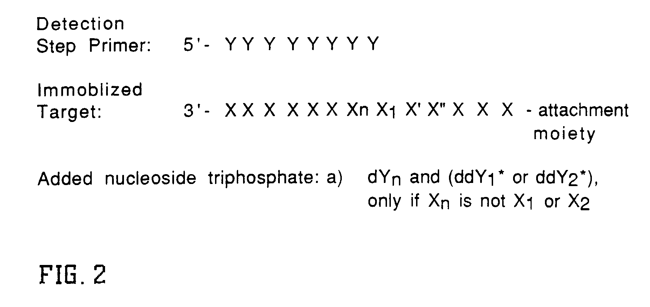

Figure 2 illustrates schematically another embodiment of the invention. The detection step primer in Fig. 2 is complementary to a portion of the target sequence starting n nucleotide residues away from the variable nucleotide to be detected. The only limitation as to the number n of nucleotide residues between the 3' end of the detection step primer and the variable nucleotide is that there must be no nucleotide residues identical to the one(s) to be detected among these n nucleotide residues. In this case the added nucleoside triphosphates comprise unlabelelled dNTPs complementary to the n nucleotides between the primer and the variable nucleotide and at least one labelled nucleoside triphosphate depending on the nucleotide variation to be detected. Two or more differently labelled ddNTPs can of course be used as earlier described in the embodiments illustrated by Figure 1 b and d.

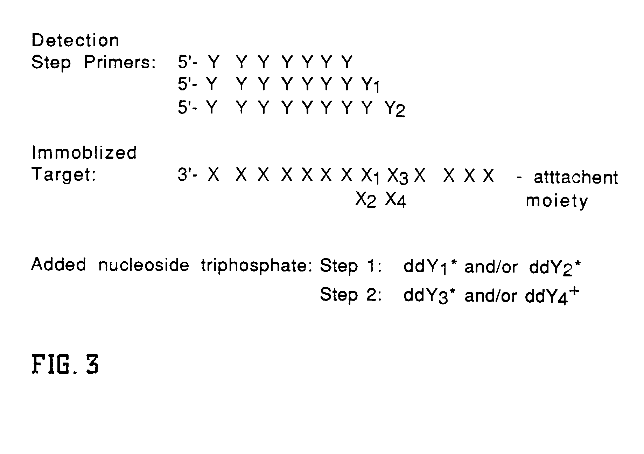

Figure 3 illustrates schematically yet another embodiment of the method according to the present invention where two or more variable nucleotide residues are identified. In this case it is necessary to design at least two different detection step primers. The first primer is selected as to be complementary to the nucleotide sequence starting immediately adjacent to the first variable nucleotide residue (primer 1 in Fig. 3). Furthermore one or two detection primers are employed spanning also the first variable nucleotide residue detected by primer 1. The test can be performed by dividing the sample into two, whereby primer 1 is added to sample 1 to identify the first variable nucleotide residue. The two other primers (primers 2 and 3 in Fig 3) are added to sample 2 to detect the second variable nucleotide. Due to the immobilization of the target nucleic acid sequence, the identification of two or more subsequent variable nucleotides can be identified from one single undivided sample, by performing the aforementioned detection steps sequentially and including an elution step after the detection of the first variable nucleotide to remove primer 1.

Reagents for use in practising the method of the invention may be provided individually or may be packaged in kit form. For example, kits might be prepared comprising one or more detection step primers and one or more labelled nucleoside triphosphates, preferably coprising also packaged combinations of one or more affinity labelled amplification primers and corresponding solid support(s).

The arrangement of the reagents within containers of the kit will depend on the specific reagents involved. Each reagent can be packaged in an individual container, but various combinations may also be possible.

The present invention is illustrated with the following examples, which are not intended to limit the scope of the invention.

### Example 1.

Identification of the apolipoprotein E polymorphism in the codons for amino acid residues 112 and 158 using [³⁵]S as label

### Synthesis of Olignucleotides

Four PCR primers (P1 -P4) and two detection step oligo primers (D1 and D2) were synthesized on an Applied Biosystems 381A DNA synthesizer. The nucleotide sequence of the oligonucleotides, and their location (given as nucleotide numbers) on the apolipoprotein E gene (Apo E) were:

P1: 5'-AAG GAG TTG AAG GCC TAC AAA T (3616 - 3637) [SEQ.ID.NO.1]
P3: 5'-GAA CAA CTG AGC CCG GTG GCG G (3649 - 3670) [SEQ.ID.NO.2]
D1: 5'-GCG CGG ACA TGG AGG ACG TG (3725 - 3744) [SEQ.ID.NO.3]
D2: 5'-ATG CCG ATG ACC TGC AGA AG (3863 - 3882) [SEQ.ID.NO.4]
P2: 5'-TCG CGG GCC CCG GCC TGG TAC A (3914 - 3893) [SEQ.ID.NO.5]
P4: 5'-GGA TGG CGC TGA GGC CGC GCT C (3943 - 3922) [SEQ.ID.NO.6]

A 5'-aminogroup was added to the primer P2 with the aminolink II reagent (Applied Biosystems). The amino group was biotinylated using sulfo-NHS-biotin (Pierce Chemical Co.) and purified by reversed phase HPLC.

### The DNA Samples

Venous blood samples were obtained from patients of known Apo E phenotype attending the Lipid Outpatient Clinic of the University Central Hospital of Helsinki, Finland. Leukocytic DNA was extracted according to standard procedures.

### Polymerase chain reaction-Amplification

The DNA (100 ng per sample) was amplified with the P1 and P4 primers (final concentration 1 µM) in 100 µl of a solution of 0.2 mM each of dATP, dCTP, dGTP, dTTP, 20 mM Tris-HCl, pH 8.8, 15 mM (NH₄)₂SO₄, 1.5 mM MgCl₂, 0.1 % Tween 20, 0.1 mg/ml gelatin and 2.5 units of Thermus aquaticus DNA-polymerase (United States Biochemical Corp.) in a DNA thermal cycler (Perkin-Elmer /Cetus) for 25 cycles of 1 min. at 96°C and 2 min. at 65°C. An aliquot (3 µl of a 1:100 dilution) of this first PCR amplification mixture was transferred to a second PCR. This was carried out at the conditions described above and directed by the biotinylated primer P2 and the primer P3.

### Affinity-capture of the Biotinylated Amplified Apo E DNA on Avidin-coated Polystyrene Particles

Five µl of a 5 % (w/v) suspension of avidin-coated polystyrene particles (0.8 µm, Baxter Healthcare Corp.) were added to an 80 µl aliquot of the second amplification mixture. The samples were kept at 20°C for 30 min. The particles were collected by centrifugation for 2 min. in an Eppendorf centrifuge and were washed once by vortexing with 1 ml of 15 mM NaCl, 1.5 mM Na-citrate (0.1 x SSC), 0.2 % sodium dodecyl sulphate (SDS), and once with 1 ml of 0.1 % Tween 20 in 0.15 M NaCl, 20 mM phosphate buffer, pH 7.5 (PBS). The particles were treated twice with 200 µl of 0.15 M NaOH for 5 min. at 20°C. The particles were then washed once with 1 ml of 0.1 % Tween 20 in 50 mM NaCl, 40 mM Tris-HCl, pH 7.5 and twice with 1 ml of 0.01 % Tween 20 in 50 mM NaCl, 40 mM Tris-HCl, pH 7.5. The suspension of particles in the last washing solution was divided into four parts, and the particles were collected by centrifugation in separate tubes.

### Identification of the Variable Nucleotides

The particles carrying the DNA fragment were suspended in 10 µl of 50 mM NaCl, 20 mM MgCl₂, 40 mM Tris-HCl, pH 7.5, containing 2 pmol of the detection step primer. The D1 oligonucleotide located immediately adjacent to the variable nucleotide number 3745 (codon 112, T or C,) was added to two of the tubes and the D2 oligonucleotide adjacent to the variable nucleotide number 3883 (codon 158, T or C) to two tubes. The oligonucleotide was annealed to the DNA template by heating the samples at 65°C for 2 min. and allowing them to cool to 20°C. One µl of 0.1 M dithiotreitol and [³⁵S]-labelled deoxynucleoside triphosphates (dNTP) and dideoxynucleoside triphosphates (ddNTP) were added to yield 1 µM concentrations each in a final volume of 15 µl as follows:
- for identification of T: [³⁵]S-dTTP (Amersham), ddCTP and ddGTP to two tubes, one in which the oligonucleotide D1 and one in which the oligonucleotide D2 had been annealed.
- for identification of C: [³⁵]S-dCTP (Amersham), ddTTP, and ddGTP to two tubes, one in which the oligonucleotide D1 and one in which the oligonucleotide D2 had been annealed.

Two µl (3U) of T7 DNA polymerase (Sequenase™, United States Biochemical Corp.) was added to each tube and the reaction was allowed to proceed for 6 min. at 42°C. The microparticles were washed at 20°C by vortexing twice with 1 ml of 0.1 x SSC, 0.2% SDS and twice with 0.1 % Tween 20 in PBS. For elution of the reaction products the particles were boiled in 200 µl of H₂O for 5 min., cooled on ice and centrifuged for 2 min. in an Eppendorf centrifuge. The eluted radioactivity was measured in a liquid scintillation counter. The result of an experiment where four samples of the phenotypes E2/E2 (T/T in codon 112, T/T in codon 158), E4/E3 (T/C in codon 112, C/C in codon 158), E2/E3 (T/T in codon 112, T/C in codon 158) and E4/E4 (C/C in codon 112, C/C in codon 158) were analyzed as described above is presented in the following table:

| Sample no. | Phenotype | Detector (codon) | Radioactivity eluted (cpm) | | Result |
|---|---|---|---|---|---|
| | | | T-reaction | C-reaction | |
| 1. | E2/E2 | D1 (112) | 18400 | 625 | T/T |
| 2. | E4/E3 | D1 (112) | 73700 | 58100 | T/C |
| 3. | E2/E3 | D1 (112) | 112000 | 1360 | T/T |
| 4. | E4/E4 | D1 (112) | 2310 | 99700 | C/C |
| 5. | No DNA | D1 (112) | 429 | 1195 | |
| | | | | | |
| 1. | E2/E2 | D2 (158) | 67000 | 7000 | T/T |
| 2. | E4/E3 | D2 (158) | 3220 | 35100 | C/C |
| 3. | E2/E3 | D2 (158) | 44600 | 26400 | T/C |
| 4. | E4/E4 | D2 (158) | 1485 | 19300 | C/C |
| 5. | No DNA | D2 (158) | 686 | 760 | |

### Conclusion

The differences in cpm-values obtained in the T- and C-reactions allowed unequivocal identification of the variable nucleotide in both codon 112 and codon 158 in all four DNA samples.

The variable nucleotide can optionally be determined as described above, but performing the second PCR with biotinylated primer P3 and primer P2. As detection step primers are then used primers complementary to the opposite strand of the ApoE gene:

D4: 5'- GTA CTG CAC CAG GCG GCC GC (3765 - 3746) [SEQ.ID.NO. 7]
D5: 5'- GGC CTG GTA CAC TGC CAG GC (3903 - 3884) [SEQ.ID.NO. 8]

### Example 2

Identification of the variable nucleotide in codon 112 of the apolipoprotein E gene using double labelling ([³H] and [³²P]) in one reaction.

### Oligonucloetides and DNA Samples

The PCR primers P1, biotinylated P2, P3 and P4, and the detection step primer D1 described in Example 1 were used in this example. The DNA was extracted from blood samples as described in Example 1.

### Polymerase chain reaction-Amplification and Affinity-capture

The PCR amplification and the affinity-capture of the amplified fragments were carried out as decribed in Example 1. In the last washing step each sample was divided into two aliquots.

### Identification of the variable nucleotide at codon 112

The particles were suspended in 10 µl of buffer (see Example 1) containing 2 pmol of the detection step primer D1, which hybridizes immedialtely 3' to the variable nucleotide in codon 112. The annealing reaction was carried out as in Example 1. One µl of 0.1 M dithitreitol was added.

The identification of the variable nucleotide (C or T) was now done by adding [³H]- and [³²P]-labelled dNTPs simultaneously to one sample. Either [³H]-dCTP and [³²P]-dTTP or [³H]-dTTP and [³²P]-dCTP (Amersham) were used. The [³H]-dNTPs were added to 1 µM concentrations. The [³²P]-dNTPs were diluted in unlabelled dNTP to yield 1 µM final concentrations and specific activities similar to those of the [³H]-dNTPs. All reactions contained 1 µM ddGTP. The final volume was 15 µl. Three units of T7 DNA polymerase was added, and the labelling and washing procedures were carried out as in Example 1. The eluted [³H] and [³²P] radioactivity in each sample was measured simulataneously in a Rackbeta 1219 scintillation counter (Pharmacia/Wallac) by setting the window for [³H] at channels 10-90 and the windows for [³²P] at channels 130-220.

The table below shows the result of an experiment, in which three samples, one of the phenotype E2/E3 (T/T in codon 112), one of the phenotype E4/E4 (C/C in codon 112) and one of the phenotype E3/E4 (T/C on codon 112) were analyzed either using [³H]-dCTP and [³²P]-dTTP or using [³H]-dTTP and [³²P]-dCTP:

| Sample | Labelled dNTPs | Radioactivity eluted (cpm) | |
|---|---|---|---|
| | | ³H;Ch,10-90 | ³²P; Ch.130-220 |
| E2/E3 | [³H]dCTP / [³²P]dTTP | 502 | 7870 |
| E4/E4 | [³H]dCTP / [³²P]dTTP | 6070 | 186 |
| E3/E4 | [³H]dCTP / [³²P]dTTP | 5120 | 5980 |
| No DNA | [³H]dCTP / [³²P]dTTP | 172 | 148 |
| | | | |
| E2/E3 | [³H]dTTP / [³²P]dCTP | 10800 | 183 |
| E4/E4 | [³H]dTTP / [³²P]dCTP | 394 | 4932 |
| E3/E4 | [³H]dTTP / [³²P]dCTP | 7800 | 5140 |
| No DNA | [³H]dTTP / [³²P]dCTP | 175 | 44 |

### Conclusion

The signals obtained allowed the identification of the variable nucleotide in codon 112 from undivided samples using two dNTPs carrying different labels. In this example only half of each sample was analyzed per reaction. Thus the other half of the sample can be used to analyze the nucleotide variation in codon 158 of the Apo E gene.

### Example 3.

Identification of the variable nucleotide in codon 158 of the apolipoprotein E gene after a single polymerase chain reaction amplification.

### Oligonucleotides and DNA samples

In this example the biotinylated primer P2 and the primer P3 were used in the PCR amplification. The detection step primer was D2 (see Example 1). The DNA was extracted from venous blood as described in Example 1.

### Polymerase chain reaction-Amplification and Affinity-capture

The DNA (100 ng per sample) was amplified with the biotinylated P2 and the P3 primer (final concentration 1 µM) at the conditions described in Example 1 with the following exception: Only one amplification process consisting of 30 cycles of 1 min. at 96°C, 1 min. at 55°C and 1 min. 72°C was carried out.

The affinity-capture on avidin-coated polystyrene particles was done as in Example 1. Each sample was divided into two parts in the last washing step.

### Identification of the variable nucleotide in codon 158

The particles were suspended in 10 µl of buffer (see Example 1) containing 2 pmol of the detection step primer D2, which hybridizes immediately 3' of the variable nucleotide in codon 158. The annealing reaction was as in Example 1. One µl of 0.1 M dithiotreitol was added. [³⁵S]-labelled dNTPs and ddNTPs were added to the T- and C-reactions as specified in Example 1. The primer extension reaction, the washing procedure and the elution of the bound radioactivity was done as described in Example 1.

Three samples with the phenotypes E2/E2 (T/T in codon 158), E2/E3 (T/C in codon 158) and E4/E3 (C/C in codon 158), respectively, were analyzed. The result of this experiment in presented in the table below:

| Sample | Radioactivity eluted (cpm) | | Result |
|---|---|---|---|
| | T-reaction | C-reaction | |
| E2/E2 | 64600 | 7746 | T/T |
| E2/E3 | 39600 | 22700 | T/C |
| E4/E3 | 5640 | 53500 | C/C |
| No DNA | 1325 | 1910 | |

### Conclusion

The method allowed correct identification of the variable nucleotide at position 158 also when the apo E DNA fragment was enriched by a single PCR with one primer pair.

### Example 4

Identification of the variable nucleotide in codon 112 of the apolipoprotein E gene with enzymatic detection.

### Oligonucleotides and DNA samples

The PCR primers P1, biotinylated P2, P3 and P4, and the detection step primer D1 described in Example 1 were used. The DNA was extracted from blood samples as described in Example 1.

### Polymerase chain reaction-Amplification

The DNA (100 ng per sample) was amplified with the primers P1 and P4 at 1 µM concentration as described in example 1. Three µl of a 1:100 dilution of this first PCR mixture was reamplified using the biotinylated P2 and the P3 primer at 0.1 µM concentration. The second PCR was carried out for 30 cycles of 1 min. at 96°C, 1 min. at 55°C and 1 min. at 72°C.

### Affinity-capture of the Amplified DNA in Avidin-coated Microtitration wells

Two 15 µl aliquots per sample of the second PCR mixture were transferred to microtitration wells (Nunc, Maxisorb), that had been coated with streptavidin by passive absorption. 30 µl of 0.1 % Tween 20 in 0.15 M NaCl, 0.1 M Tris-HCl, pH 7.5 (TBS) was added to each well. The microtitration strips were incubated for 3 hours at 37°C with gentle shaking. The wells were washed three times with 200 µl of 0.1 % Tween 20 in TBS at 20°C. The wells were then treated twice with 100 µl of 50 mM NaOH for 5 min. at 20°C followed by washing twice with 200 µl of 0.1 x SSC, 0.2 % SDS, twice with 0.1 % Tween 20 in TBS, once with 0.1 % Tween 20 in 50 mM NaCl, 40 mM Tris-HCl, pH 7.5, and finally once with 0.01 % Tween 20 in 50 mM NaCl, 40 mM Tris-HCl, pH 7.5.

### Identification of the variable nucleotide

Ten pmol of the oligonucleotide D1 was added to each well in 50 µl of 0.9 M NaCl, 0.2 M Tris-HCl, pH 7.5. The wells were heated at 65°C for 2 min. and allowed to cool slowly to 20°C. The mixture was discarded and the wells were washed once with 200 µl of 0.25 M NaCl, 0.2 M Tris-HCl, pH 7.5 at 20°C. 50 µl of a solution consisting of 1 µM digoxigenin-11-dUTP (Boehringer-Mannheim), 1 µM ddCTP, 1 µM ddGTP, 0.2 µM oligonucleotide D1, 6 mM dithiotreitol, 37.5 mM NaCl, 15 mM MgCl₂, 30 mM Tris-HCl, pH 7.5 and 3 units of T7 DNA polymerase was added. The microtitration strips were incubated at 42°C for 10 min. and the wells were washed twice with 200 µl of 0.1 x SSC, 0.2 % SDS, and three times with 200 µl of 0.1 % Tween 20 in TBS. Then 60 µl of a 1:500 dilution of an anti-digoxigenin-alkaline phosphtase conjugate (Boehringer-Mannheim) in a solution of 0.1 % Tween 20, 1 % bovine serum albumin in TBS was added, and the microtitration strips were incubated at 37°C for 2 hours with gentle shaking. The wells were washed six times with 0.1 % Tween 20 in TBS and once with 1M diethanolamine-0.5 M MgCl₂ buffer, pH 10. Finally 60 µl of 2 mg/ml p-nitrophenyl phosphate in the alkaline buffer was added. After development of colour for 20 min. at room temperature 100 µl of the alkaline buffer was added and the absorbance of the formed product was measured at 405 nm in a spectrophotometric reader.

Two samples with the phenotypes E2/E2 (T/T in codon 112) and E4/E4 (C/C in codon 112) were analyzed. The result of this experiment is presented in the table below:

| Sample | Absorbance at 405 nm (duplicate samples) | | Result |
|---|---|---|---|
| E2/E2 | 1.180 | 0.707 | T/T |
| E4/E4 | 0.040 | 0.010 | C/C |
| No DNA | 0.025 | 0.010 | |

### Conclusion

The variable nucleotide in codon 112 was identified after incorporation of digoxigenin-11-dUTP and subsequent detection with an antibody labelled with alkaline phosphatase.

### Example 5.

Identification of the apolipoprotein E polymorphism in the codon for amino acid residue 112 using fluorescent labels

### Oligonucleotides and DNA samples

The PCR primers P1, biotinylated P2, P3 and P4, and the detection step primer D1 described in Example 1 are used. The DNA is extracted from blood samples as described in Example 1.

### Polymerase chain reaction-Amplification and Affinity-capture

The DNA (100 ng per sample) is amplified with the primers P1 and P4 at 1 µM concentration as described in Example 1. Three µl of a 1:100 dilution of this first PCR mixture is reamplified using the biotinylated P2 and the P3 primer at 1 µM concentration. The second PCR is carried out for 25 cycles of 1 min. at 96°C, 1 min. at 55°C and 1 min. at 72°C. The biotinylated amplified DNA fragments are captured on avidin-coated polystyrene particles as in Example 1. Each sample is divided into two parts in the last washing step.

### Identification of the Variable Nucleotide in Codon 112

The particles carrying the amplified DNA are suspended in 10 µl of buffer (see Example 1) containing 5 pmol of the detection step primer which hybridizes immediately 3' of the variable nucleotide in codon 112. The annealing reaction is carried out as in Exmple 1. One µl of 0.1 M dithiotreitol is added to each tube. For identification of T, 400 pm of fluorescent ddTTP (T-terminator; DuPont, NEK-528T) is added to one of the tubes. For identification of C, 40 pm of fluorescent ddCTP (C-terminator; DuPont, NEK-519C) is added to the other tube. Five units of T7 DNA polymerase is added to both tubes to a final reaction volume of 15 µl. The reaction is allowed to proceed for 5 min. at 37°C. The particles are washed as described in Example 1 and the reaction products are eluted. The fluorescence of the eluant is measured in a fluorescence spectrophotometer (Merck/Hitatchi, F-1000) using 490 nm as the excitation wavelength and 550 nm for measurement of the emitted fluorescence.

### Interpretation of the Result

A positive signal from only the T-reaction shows that the subject is homozygous for a cystein residue at position 112.

A positive signal from only the C-reaction shows that the subject is homozygous for an arginine residue at position 112.

Positive signals from both reactions show that the subject is heterozygous, ie. has one allele with a cystein residue, and one allele with an arginine residue at position 112 of the apolipoprotein E gene.

### Example 6.

Detection of the sickle cell muatation in the sequence encoding codon 6 of the human β-globin gene.

### Synthesis of Oligonucleotides

The PCR primers are designed to contain 3'-ends that are mismatched to the otherwise strongly homologuous δ-globin gene. Two PCR primers, denoted B1 and B2, and one detection step primer B3, are synthesised by the method described in Example 1. The primer B2 is biotinylated as described in Example 1. The nucleotide sequence of the oligonucleotides and their location on the β-globin gene (as nucleotide numbers relative to the transcription intiation site) are the following:

B1: 5′-CAT TTG CTT CTG ACA CAA CT (-49 - -30) [SEQ.ID.NO. 9]
B3: 5′-CAT GGT GCA CCT GAC TCC TG (-1 - 19) [SEQ.ID.NO. 10]
B2: 5′-CAA CTT CAT CCA CGT TCA CC (73 - 54). [SEQ.ID.NO. 11]

### Polymerase chain reaction-Amplification and Affinity-capture

The DNA is extracted from blood samples as described in Example 1. The DNA (100 ng per sample) is amplified with the primers B1 and biotinylated B2 as described in Example 3. The biotinylated amplified DNA fragments are captured on avidin-coated polystyrene particles as in Example 1. Each immobilized sample is divided into two parts.

### Identification of the A → T Mutation in Codon 6

The particles carrying the amplified DNA sample are suspended in 10 µl of buffer (see Example 1) containing 2 pmol of the B3 detection step primer, which hybridizes immediately 3' of the mutation site in codon 6. The annealing reaction is carried out as in Exmple 1. One µl of 0.1 M dithiotreitol is added. [³⁵S]-labelled dNTPs and ddNTPs are added to yield 0.2 µM concentrations in a final volume of 15 µl as follows:
- for identification of the normal allele (A): [³⁵S]-dATP, ddTTP, ddGTP to one of the tubes
- for identification of the mutation (T): [³⁵S]-dTTP, ddATP, ddGTP to the other tube.

One unit of T7 DNA polymerase is added, and the reaction is allowed to proceed for 5 min. at 37°C. The particles are washed, the reaction products are eluted and the eluted radioactivity is measured in a scintillation counter as described in Example 1.

### Interpretation of the Result

A positive signal from only the A-reaction shows that the subject is homozygous and normal.

A positive signal from only the T-reaction shows that the subject is homozygous for the sickle cell mutation.

A positive signal from both reactions show that the subject carries the sickle cell mutation in one allele ie. is heterozygous.

The variable nucleotide can optionally be determined as described above, but performing the PCR with biotinylated primer B1 and primer B2. As detection step primer is then used a primer complementary to the opposite strand of the β-globin gene:

B4: 5′- CAG TAA CGG CAG GCG GCC GC (40 - 21) [SEQ.ID.NO. 12]

### Example 7.

Identification of the ΔF508 deletion in the cystic fibrosis.

### Synthesis of oligonucleotide primers

Two amplification primers (CF1 and CF3) and one detection step primer (CF2) were synthesises as described in Example 1. The primers were designed based on the known nucleotide sequence of the CF gene (Riordan et al., Science 1989; 245: 1066-1072). The sequence and the position on the CF gene of the primers were:

CF1: 5'-CTG GAG CCT TCA GAG GGT AAA AT-3' (1555-77) [SEQ.ID.NO.13]
CF2: 5'-TGG CAC CAT TAA AGA AAA TAT CAT-3'(1629-52) [SEQ.ID.NO.14]
CF3: 5'-CAT GCT TTA ATG ACG CTT CTA TA-3' (1703-81) [SEQ.ID.NO.15]

The primer CF3 was biotinylated as described in Example 1.

### The DNA samples

The target DNA was extracted from leukocyte cells from finnish cystic fibrosis patients, who had been clinically charactherized in regard of the ΔF508 mutation, according to standard methods.

### PCR-Amplification and Affinity-capture of the Target DNA

The DNA (100 - 200 ng per sample) was amplified with the biotinylated CF3 and the CF1 primer (final concentrations 0.15 µM and 0.6 µM, respectively) at the conditions decribed in Example 1, with the following exception: Only one amplification process consisting of 31 cycles of 1 min at 96°C, 1 min at 60°C and 1 min at 72°C was carried out.

The affinity capture on steptavidin-coated microtitration wells was performed as described in Example 4.

### Identification of the variable nucleotide

The detection step primer extension was performed as described in 50 µl of 50 mM Tris-HCl, pH 8.8, 1.5 mM MgCl₂, 15 mM (NH₄)₂SO₄, 0.1% Tween 20, 0.01% gelatin containing 0.2 µM of the primer CF2 and 2 units of Taq DNA polymerase. For identification of T, 1 µCi ³H-dTTP (Amersham) and 0.8 µM ddGTP, and for identification of C, 2 µCi ³H-dCTP, 0.8 µM ddTTP were added in two parallel wells and the plates were incubated for 10 min at 55°C. The wells were washed three times with 200 µl of 0.1 % Tween 20 in TBS buffer and the incorporated label was eluted with 60 µl of 50 mM NaOH for 5 min at room temperature and measured in a liquid scintillation counter.

Three samples with the genotypes C/C, T/T and T/C in nucleotide 1653 of the CFTR-gene, i.e. one normal homozygote, one ΔF508 homozygote and one heterozygote, were used in this example. These samples had been previously genotyped by other methods (Kere et al, Hum. Genet., 1990; 85:413-415). The results in the table below show that the identification of each genotype is clear.

| Sample | ³H-dTTP (cpm) | ³H-dCTP (cpm) | cpmC/cpmT | Genotype^{x)} |
|---|---|---|---|---|
| Normal homozygote | 349 | 9832 | 28.2 | C/C |
| | | | | |
| ΔF508 homozygote | 11539 | 52 | 0.005 | T/T |
| | | | | |
| Heterozygote | 11358 | 7457 | 0.66 | T/C |

| | | | | |
|---|---|---|---|---|
| x) nucleotide 1653 of the CFTR gene. | | | | |

### Conclusion

The genotypes of the samples were verified according tp cpmC/cpmT ratios after incorporation of ³H-dTTP and ³HdCTP.

### Example 8.

Detection of point mutations in the sequence encoding codon 12 in the K-ras gene.

### Synthesis of oligonucleotides

Two PCR primers, denoted R1 and R2, respectively, are synthesised and the primer R1 is biotinylated as described in Example 1. For detection of a mutation of the glycine residue (encoded by GGT) in position 12, two detection step primers, R3 and R4, are synthesised. R3 is used to detect a mutated G in the second position and R4 to detect a mutated G in the first position of codon 12. The sequence and position (as nucleotide numbers) of the oligonucleotides on the first exon of the K-ras gene is given below:

R1: 5'-ATG ACT GAA TAT AAA CTT GTG (1 - 20) [SEQ.ID.NO. 16]
R2: 5'-TTC GTC CAC AAA ATG ATT CTG (94 - 74) [SEQ.ID.NO. 17]
R3: 5'-AAG GCA CTC TTG CCT ACG CCA (56 - 36) [SEQ.ID.NO. 18]
R4: 5'-AGG CAC TCT TGC CTA CGC CAC (55 - 35) [SEQ.ID.NO. 19]

### Polymerase chain reaction-Amplification, Affinity-capture and Annealing of the Detection step Primers

The DNA is extracted from tumor cell samples by standard methods using digestion with proteinase K, phenol extraction and precipitation with ethanol. 100 ng of the purified DNA is amplified with the biotinylated primer R1 and the primer R2 using the conditions decribed in Example 3, except that the primer annealing temperature is 50°C. The amplified DNA is captured on avidin-coated polystyrene particles, denatured and the particles are washed as described in Example 1. The immobilized DNA sample is divided into two tubes. The particles in one of the tubes are suspended in 10 µl of buffer (see Example 1) containing 2 pmol of the oligonucleotide R3, which will anneal immediately 3' of the C complementary to the second G in codon 12. In the other tube 10 µl of buffer containing 2 pmol of the oligonucleotide R4 annealing 3' to the C complementary to the first G in codon 12 is used. The annealing reaction is carried out as in Example 1. One µl of 0.1 M dithiotreitol is added. The mutation in codon 12 is analyzed according to method A or method B below.

### A. Identification of a gly → non-gly Mutation in Codon 12

A mixture of [³⁵S]-dATP, [³⁵S]-dGTP, [³⁵S]dTTP and ddCTP are added to both tubes to give a final concentration of 1µM each in 15 µl. One unit of T7 DNA polymerase is added and the reaction is allowed to proceed for 5 min. at 37°C. The particles are washed, the reaction products are eluted and the eluted radioactivity is measured in a scintillation counter as described in Example 1.

A positive signal from the tube, in which R3 had been annealed, shows that at least a part of the K-ras genes in the sample has mutated to a valine (encoded by GTT), an aspartic acid (GAT) or an alanine (GCT) residue in position 12.

A positive signal from the tube, in which R4 had been annealed, shows that there is a cystein (TGT), a serine (AGT) or an arginine (CTG) residu in position 12 in at least a part of the K-ras genes.

Lack of signal from both tubes shows that codon 12 in both alleles is the normal GGT encoding a glycine residue.

### B. Characterization of the Mutation in Codon 12

The exact characterization of the mutation is done by adding to both tubes a mixture of [³²P]-dATP, [³⁵S]-dGTP, [³H]-dTTP and ddCTP to a final concentration of 1 µM in 15 µl. The [³²P]-dATP and the [³⁵S]-dGTP are diluted in unlabelled dATP and dGTP, respectively, to yield similar specific activities as that of the [³H]-dTTP (about 100 Ci/mmol). The difference in scintillation counting efficiency between the three radioisotopes is taken into account to design a reaction mixture, which will give equal cpm values in the channels used for measurement (see below).

One unit of T7 DNA polymerase is added and the reaction is allowed to proceed for 5 min. at 37°C. The particles are washed and the reaction products are eluted as described in Example 1.

The radioactivity emitted by [³H], [³⁵S] and [³²P] in the eluted product is measured simultaneously in a scintillation counter. In a Rackbeta 1219 counter (Pharmacia/Wallac) the following window settings are used: for measurement of [³H]: channels 10-90, for measurement of [³⁵S] channels 95-145 and for measurement of [³²P] channels 170-220. Before interpretation of the result corrections for the overflow of signal from the [³⁵S] to the [³H] channels (24 %) and from the [³²P] to the [³⁵S] channels (13 %) are done.

The results are interpreted as specified in the table below:

| Detection step primer | Signal from | | | Result | |
|---|---|---|---|---|---|
| | ³H | ³⁵S | ³²P | Codon 12 | Amino acid |
| R3 | + | - | - | GAT | aspartic acid |
| R3 | - | + | - | GCT | alanine |
| R3 | - | - | + | GTT | valine |
| R3 | - | - | - | GGT | glycine |
| | | | | | |
| R4 | + | - | - | AGT | serine |
| R4 | - | + | - | CGT | arginine |
| R4 | - | - | + | TGT | cystein |
| R4 | - | - | - | GGT | glycine |

The mutations in codon 12 of the K-ras gene can optionally be determined as described above, but performing the PCR with biotinylated primer R2 and primer R1. As detection step primer is then used a primer complementary to the opposite strand of the K-ras gene:

R5: 5'- AAC TTG TGG TAG TTG GAG CT  (14 - 33)  [SEQ.ID.NO. 20]
R6: 5'- ACT TGT GGT AGT TGG AGC TG  ( 15 - 34) [SEQ.ID.NO. 21]

### Example 9

Detection of point mutations in the sequence encoding codon 12 in the N-ras gene in the presence of non-mutated cells

### Synthesis of oligonucleotides

Two PCR primers, denoted X1 and X2, respectively, were synthezised and the primer X1 is biotinylated as described in Example 1. For detection of the mutation of the second nucleotide in codon 12 (G replaced by A) a detection step primer X3 is synthesized. The sequence and position (as nucleotide numbers) of the oligonucleotides on the first exon of the N-ras gene is given below:

X1: 5'-GAC TGA GTA CAA ACT GGT GG  (3 - 22)  [SEQ.ID.NO. 22]
X2: 5'-CTC TAT GGT GGG ATC ATA TT  (111-91)  [SEQ.ID.NO. 23]
X3: 5'-ACT GGT GGT GGT TGG AGC AG  (15 - 34) [SEQ.ID.NO. 24]

### The DNA samples

A cell line known to harbour a nucleotide transition (G → A) in the second position of codon 12 of the N-ras gene (cell line PA-1, ATCC CRL1572) was used as model system to mimic a situation where cell samples from AML patients, could be analyzed for minimal residual mutated cells during follow up of the treatment of the patients.

DNA from PA-1 cells and from normal human lymphocytes was extracted by standard methods as in Example 7 and mixed to yield a series of samples representing 100% to 0.1% of mutated cells.

### Polymerase chain reaction-Amplification

The extracted DNA (100 ng per sample) was amplified using primers X1 and X2 at conditions described in Example 3.

### Affinity-capture on streptavidin-coated magnetic partices and Annealing of the Detection Step Primers

Eighty µl of the amplification mixture and 20 µl of 1 M NaCl was added to 300 µg of streptavidin-coated magnetic polystyrene beads (Dynabeads^{R} M-280, Strepatvidin, Dynal AS). The samples were kept for 30 min at 20°C, the beads were separated from the reaction mixture using a magnetic particle concentrator (MPC-E, Dynal AS) and the mixture was discarded. The beads were washed three times with 1 ml of 0.5 M NaCl in 20 mM sodium phosphate buffer, pH 7.5, 0.1% Tween 20 and treated twice with 100 µl of 50mM NaCl, 20 mM MgCl₂, 40 mM Tris-HCl, pH 7.5, containing 2 pmol of detection step primer, X3. The primer was allowed to anneal to the DNA template for 10 min at 37°C. One µl of 0.1 M dithiotreitol, 15 pmol of 3H-labelled dTTP and 1 unit of T7 DNA-polymerase (United Stated Biochemical Corporation) were added to yield a final volume of 15 µl. The reaction was allowed to proceed for 5 min at 37°C. The beads were washed 3 times and treated with 100 µl of 50 mM NaOH, 0.15 M NaCl for 5 min at 20°C. The eluted radioactivity was measured in a liquid scintillation counter. The results are given in the table below:

| Detection of minority point mutations in PA-1 cells | |
|---|---|
| Mutated cells (%) | ³H incorporated (cpm)* |
| 50 | 23,000 |
| 5 | 3,400 |
| 0.5 | 800 |
| 0 | 490 |

| | |
|---|---|
| *) Incorporation of ³H corresponding to A in codon 12 (GAT) | |

### Conclusion

The result shows that the method according present invention detects as littes as 0.25% of mutated monoallelic N-ras DNA from a background of normal DNA. This sensitivity is well in the range needed to screen AML patients and to monitor the identified mutations during treatment and follow up of the disease.

### Example 10

Detection of point mutation (A - T) in the sequence encoding codon 215 (amino acid sequence number is relative to the NH2 terminal proline) of HIV-1 reverse transcriptase gene

### Synthesis of oligonucleotides

Two PCR primers (H1 and H2) and one detection step primer (H3) are synthesized by the method described in Example 1. The primer H2 is biotinylated as described in Example 1. The nucleotide sequences of the oligonucleotides and their location on the HIV-1 reverse transcriptase gene (nucleotide numbering is according to Ratner et al., 1985, Nature 313: 277-281) are the following:

H1: 5′ -GAG AAT CCA TAC AAT ACT CCA  (2286-2306) [SEQ.ID.NO. 25]
H2: 5′ -TAA CCC ATC CAA AGG AAT GGA  (2824-2804) [SEQ.ID.NO. 26]
H3: 5′ -ATC TGT TGA GGT GGG GAC TT  (2752-2771) [SEQ.ID.NO. 27]

### Polymerase chain reaction-Amplification and Affinity-capture

The DNA is extraxted from HIV-1 infected cells as described in Example 1. The DNA (100 ng per sample) is amplified with the primers H1 and H2 (final concentration 0.2 mM) as described in Example 1 with the exception that the primer annealing temperature is 50° C. The biotinylated amplified DNA fragments are captured on streptavidin-coated microtiter plate wells as described in Example 4. Each sample is bound to two parallel wells. The DNA fragments bound to the microtiter plate walls are denatured and washed as described in Example 4.

### Identification of the A - T mutation in codon 215

The annealing of the detection primer H3 and the detection reaction are done simultaneously at 50° C for 10 min in 50 mM Tris-HCl, pH 8.8, 1.5 mM MgCl₂, 15 mM (NH₄)₂SO₄, 0.1 % Tween 20, 0.01 % gelatin and 0.2 µM primer H3 containing for A reaction: 1 µCi of ³H-dATP (82 Ci/mmol, Amersham, UK), 0.8 µM of ddTTP and ddCTP and 1 U of Taq DNApolymerase (Promega). For T-reaction: 1 µCi of ³H-dTTP (98 Ci/mmol) and 0.8 µM of ddATP and ddCTP. The wells are washed three times with 0.1 % Tween 20 in TBS and the radioactivity is eluted with 60 ml of 50 mM NaOH for 5 min at room temperature. The eluted radioactivity is measured in liquid scintillation counter.

### Interpretation of results

A positive signal from only the A-reaction shows that the virus isolate is wild type. A positive signal from only the T-reaction shows that the virus isolate carries the mutation of the first nucleotide of codon 215. Consequently, the amino acid Thr has changed to Phe or Tyr. Positive reactions from both reactions show that the virus is a mixed population of wild type and mutant viruses.

The variable nucleotides in codons 67 and 70 could be determined in a similar way from the same amplification product.

## Claims

1. A method for detecting a specific nucleotide variation at a defined site in a target nucleic acid polymer wherein a first nucleotide residue is replaced by a second nucleotide residue, comprising the steps of:
a) performing a modified amplification reaction in which one of the two amplification primers comprises a first attachment moiety bound to the primer, thereby obtaining a double-stranded amplification product in which only one of the strands comprises a first attachment moiety, where said first attachment moiety is one half of an affinity pair chosen from the group of biotin-avidin/streptavidin, antigens/haptens-antibodies or heavy metal derivatives-thiogroups;
b) sequentially in any order rendering the amplification product obtained in step a) single-stranded and immobilizing the strand comprising the first attatchment moiety to a solid support with the aid of the other component of the affinity pair, whereafter all unbound material is removed:
c) hybridising a detectable amount of the immobilised single-stranded target nucleic acid obtained in step b) with a detection step primer comprising a plurality of nucleotide residues, said primer being complementary to the nucleotide sequence of interest in a region disposed toward the 3' end from the defined site such that when the primer is hybridised to the target nucleic acid there are no nucleotide residues between the defined site and the 3' end of the detection step primer that are identical to the first or second nucleotide residues to be detected;
d) extending the primer using a polymerizing agent in a mixture comprising one or more nucleoside triphosphates wherein the mixture includes at least one nucleoside triphosphate complementary to either the first or second nucleotide residue which comprises means for detecting the incorporation of the nucleoside triphosphate in a nucleic acid polymer, and optionally one or more chain terminating nucleoside triphospates; and
e) detecting the incorporation of the nucleoside triphosphate using said means, whereby the identity of the nucleotide residue at the defined site is determined.

2. A method for detecting a plurality of specific nucleotide variations at defined sites in a target nucleic acid polymer wherein at least a first nucleotide residue is replaced by a second nucleotide residue at a first defined site and a third nucleotide is replaced by a fourth nucleotide residue at a second defined site wherein the first nucleotide variation is detected by applying the method of claim 1, and subsequent nucleotide variations are detected after removal of the extended first detection step primer by applying steps c) d) and e) of the method of claim 1 to the already immobilized strand for each of the other nucleotide variations.

3. A method of detecting in a patient a predisposition to a genetic disorder resulting from a specific nucleotide variation at a defined site in a genetic material of the patient, wherein a first nucleotide residue is replaced by a second nucleotide residue, **characterized** by applying the method of claim 1 for detecting said variation on a sample containing a detectable amount of genetic material derived from the patient.

4. A method for detecting the existence of point mutations at a defined site in the genome of a microorganism leading to altered patogenicity or resistance to therapy in the microorganism, wherein a first nucleotide is replaced by a second nucleotide residue, **characterized** by applying the method of claim 1 for detecting said pont mutation on a sample containing a detectable amount of genetic material derived from the microorganism.

5. A method for detecting cells having a point mutation at a defined site in the genetic material, wherein a first nucleotide residue is replaced by a second nucleotide residue, when said cells are mixed in a cell population, **characterized** by applying the method of claim 1 for detecting said point mutation on detectable quantity of genetic material from the cell population in which the ratio of mutated to unmutated cells is maintained.

6. A method according to claims 1-5, wherein the detection step primer is complementary to a region of the nucleotide sequence of interest extending toward the 3' end of the target nucleic acid polymer from the nucleotide residue immediately adjacent to the defined site.

7. A method according to claims 1-5, wherein the detection step primers are immobilized during or after the detection step primer extension step d).

8. A method according to claims 1-5, wherein the nucleoside triphosphate comprising means for detecting the incorporation of the nucleoside triphosphate in a nucleic acid is a deoxyucleoside triphosphate.

9. A method according to claims 1-5, wherein the nucleoside triphosphate comprising means for detecting the incorporation of the nucleoside triphosphate in a nucleic acid polymer is a dideoxynucleoside triphosphate.

10. A method according to claims 1-5, wherein the mixture includes a second nucleoside triphosphate comprising a second means, different from said first means, for detecting the incorporation of the second nucleoside triphosphate in a nucleic acid polymer.

11. A method according to claim 1, wherein the extended product of step (d) is eluted before determining the incorporation of the incorporated nculeoside triphosphate.

12. A method according to claim 7, wherein the nucleotide variations are detected in one single step by adding a plurality of detection step primers and differently labelled nucleoside triphospates identifying the variable nucleotide residues.

13. A method according to claim 4, wherein the microorganism is HIV.

14. A method according to claim 13, wherein the point mutation is at a site selected from among Asp 67, Lys 80 and Thr 215.

15. A method according to claim 5, wherein the cells are lymphocytes.

16. A method according to claim 15, wherein the cells are leukemic cells.

17. A kit for use in determining specific nucleotide variations in a target nucleic acid polymer comprising in packaged combination
(a) one pair of amplification oligonucleotide primers where one primer is complementary to and hybridizes with a portion of one of the strands of the double-stranded target nucleic acid polymer and the other is complementary to and hybridizes to a portion of the other strand of said target nucleic acid and also comprises a first attachment moiety, where said first attachment moiety is one half of an affinity pair chosen from the group of biotin-avidin/streptavidin, antigens/haptens-antibodies or heavy metal derivatives-thiogroups, and which pair of oligonucleotide primers is effective as primers for enzymatic nucleic acid polymerization;
b) at least one detection step primer comprising an oligonucleotide which is complementary to and hybridizes with a portion 3' to a variable nucleotide of the target nucleic acid polymer; and optionally
c) at least one solid support comprising a solid matrix and at least one attachment site which is capable of immobilizing the oligonucleotide of the amplification probe through the first attachment moiety; and
d) at least one nucleoside triphosphate containing means for detecting the incorporation of the nucleoside triphosphate in a nucleic acid polymer.

18. A kit according to claim 17 for use in the identification of the nucleotide variation of apolipoprotein E polymorphism, wherein the detection step primer comprises the sequence 5'-GCG CGG ACA TGG AGG ACG TG.

19. A kit according to claim 17 for use in the identification of the nucleotide variation of apolipoprotein E polymorphism, wherein the detection step primer comprises the sequence 5'-ATG CCG ATG ACC TGC AGA AG.

20. A kit according to claim 17 for use in the identification of the nucleotide variation of apolipoprotein E polymorphism, wherein the detection step primer comprises the sequence 5'-GTA CTG CAC CAG GCG GCC GC.

21. A kit according to claim 17 for use in the identification of the nucleotide variation of apolipoprotein E polymprhism, wherein the detection step primer comprises the sequence 5'-GGC CTG GTA CAC TGC CAG GC.

22. A kit according to claim 17 for use in the detection of the nucleotide variation in codon 6 of the human b-globin gene causing sickle cell anemia, wherein the detection step primer comprises the sequence 5'CAT GGT GCA CCT (GAC TGG TG.

23. A kit according to claim 17 for use in the detection of the nucleotide variation in codon 6 of the human b-globin gene causing sickle cell anemia, wherein the detection step primer comprises the sequence 5'-CAG TAA CGG CAG GCG GCC GC.

24. A kit according to claim 17 for use in the detection of a nucleotide variation in codon 12 of the K-ras gene. wherein the detection step primer comprises the sequence 5'-AAG GCA CTC TTG CCT ACG CCA.

25. A kit according to claim 17 for use in the detection of a nucleotide variation in codon 12 of the K-ras gene, wherein the detection step primer comprises the sequence 5'-AGG CAC TCT TGC CTA CGC CAC.

26. A kit according to claim 17 for use in the detection of a nucleotide variation in codon 12 of the K-ras gene, wherein the detection step primer comprises the sequence 5'-AAC TTG TGG TAG TTG GAG CT.

27. A kit according to claim 17 for use in the detection of a nucleotide variation in codon 12 of the K-ras gene, wherein the detection step primer comprises the sequence 5'-ACT TGT GGT AGT TGG AGC TG.

28. A kit according to claim 17 for use in the detection of a nucleotide variation in codon 12 of the N-ras gene, wherein the detection step primer comprises the sequence 5'-ACT GGT GGT GGT TGG AGC AG.

29. A kit according to claim 17 for use in the detection of resistance to AZT in HIV-1 viruses, wherein the detection step primer comprises the sequence 5'-ATC TGT TGA GGT GGG GAC TT.

30. A kit according to claim 17 for use in the detection of cystic fibrosis, wherein the detection step primer comprises the sequence 5'-TGG CAC CAT TAA AGA AAA TAT CAT.

## Patentansprüche

1. Verfahren zum Nachweis einer spezifischen Nucleotidvariation an einer bestimmten Stelle in einem ZielNucleinsäurepolymer, in dem ein erster Nucleotidrest durch einen zweiten Nucleotidrest ersetzt ist, wobei das Verfahren folgende Schritte umfaßt:
(a) Durchführung einer modifizierten Amplifikationsreaktion, in der einer der zwei Amplifikationsprimer eine erste, an den Primer gebundene Verknüpfungseinheit umfaßt, wobei ein Amplifikationsprodukt in Doppelstrangform erhalten wird, in dem nur einer der Stränge eine erste Verknüpfungseinheit umfaßt, wobei die erste Verknüpfungseinheit eine Hälfte eines Affinitätspaares ist, ausgewählt aus Biotin-Avidin/Streptavidin, Antigene/Haptene-Antikörper oder Schwermetallderivate-Thiogruppen;
(b) in beliebiger Reihenfolge, Umwandeln des in Schritt (a) erhaltenen Amplifikationsproduktes in Einzelstrangform und Immobilisieren des die erste Verknüpfungseinheit umfassenden Strangs an einen festen Träger mit Hilfe anderer Komponenten des Affinitätspaares, worauf alles ungebundene Material entfernt wird;
(c) Hybridisierung einer nachweisbaren Menge der immobilisierten Ziel-Nucleinsäure in Einzelstrangform, die in Schritt (b) erhalten wurde, mit einem Nachweisschritt-Primer, umfassend eine Vielzahl von Nucleotidresten, wobei der Primer komplementär zur Nucleotidsequenz von Interesse in einem Bereich in Richtung 3'-Ende von der bestimmten Stelle aus ist, so daß, wenn der Primer mit der Zielnucleinsäure hybridisiert, es keine Nucleotidreste zwischen der bestimmten Stelle und dem 3'-Ende des Nachweisschritt-Primers gibt, die identisch mit den ersten oder zweiten nachzuweisenden Nucleotidresten sind,
(d) Verlängerung des Primers unter Verwendung eines Polymerisationsmittels in einem Gemisch, das eines oder mehrere Nucleosidtriphosphate umfaßt, wobei das Gemisch mindestens ein Nucleosidtriphosphat umfaßt, das komplementär zum ersten oder zweiten Nucleotidrest ist, umfassend Mittel zum Nachweis des Einbaus des Nucleosidtriphosphats in ein Nucleinsäurepolymer, und gegebenenfalls eines oder mehrere Kettenabbruch-Nucleosidtriphosphate; und
(e) Nachweis des Einbaues des Nucleosidtriphosphats unter Verwendung der Mittel, wobei die Identität des Nucleotidrestes an der bestimmten Stelle festgestellt wird.

2. Verfahren zum Nachweis einer Vielzahl von spezifischen Nucleotidvariationen an bestimmten Stellen in einem Ziel-Nucleinsäurepolymer, wobei mindestens ein erster Nucleotidrest durch einen zweiten Nucleotidrest an einer ersten definierten Stelle ersetzt wird und ein drittes Nucleotid durch einen vierten Nucleotidrest an einer zweiten definierten Stelle ersetzt wird, wobei die erste Nucleotidvariation nachgewiesen wird, indem man das Verfahren von Anspruch 1 anwendet, und die nachfolgenden Nucleotidvariationen nachgewiesen werden, nachdem der verlängerte Primer des ersten Nachweisschrittes entfernt wurde, indem Schritte (c), (d) und (e) des Verfahrens nach Anspruch 1 auf den schon immobilisierten Strang für jede der anderen Nucleotidvariationen angewendet wird.

3. Verfahren zum Nachweis einer Prädisposition für eine genetische Erkrankung in einem Patienten, die von einer spezifischen Nucleotidvariation an einer bestimmten Stelle im genetischen Material des Patienten herrührt, wobei ein erster Nucleotidrest durch einen zweiten Nucleotidrest ersetzt ist, gekennzeichnet durch die Anwendung des Verfahrens nach Anspruch 1 zum Nachweis der Variation an einer Probe, die eine nachweisbare Menge des vom Patienten stammenden genetischen Materials enthält.

4. Verfahren zum Nachweis des Vorhandenseins von Punktmutationen an einer bestimmten Stelle im Genom eines Mikroorganismus, die zu einer veränderten Pathogenität oder Therapieresistenz in dem Mikroorganismus führen, wobei ein erstes Nucleotid durch einen zweiten Nucleotidrest ersetzt ist, gekennzeichnet durch die Anwendung des Verfahrens nach Anspruch 1 zum Nachweis der Punktmutation an einer Probe, die eine nachweisbare Menge des vom Mikroorganismus stammenden genetischen Materials enthält.

5. Verfahren zum Nachweis von Zellen mit einer Punktmutation an einer bestimmten Stelle im genetischen Material, wobei ein erster Nucleotidrest durch einen zweiten Nucleotidrest ersetzt ist, wenn die mutierten Zellen in einer Zellpopulation gemischt vorliegen, gekennzeichnet durch die Anwendung des Verfahrens nach Anspruch 1 zum Nachweis der Punktmutation an einer nachweisbaren Menge des genetischen Materials von der Zellpopulation, in der das Verhältnis mutierter zu nicht mutierten Zellen erhalten ist.

6. Verfahren nach einem der Ansprüche 1 bis 5, wobei der Nachweisschritt-Primer komplementär ist zu einem Bereich der Nucleotidsequenz von Interesse, der sich in Richtung 3'-Ende des Ziel-Nucleinsäurepolymers vom unmittelbar neben der bestimmten Stelle gelegenen Nucleotidrest aus erstreckt.

7. Verfahren nach einem der Ansprüche 1 bis 5, wobei die Nachweisschritt-Primer während oder nach dem Verlängerungsschritt (d) des Nachweisschritt-Primers immobilisiert werden.

8. Verfahren nach einem der Ansprüche 1 bis 5, wobei das Nucleosidtriphosphat, das das Mittel zum Nachweis des Einbaus des Nucleosidtriphosphats in eine Nucleinsäure enthält, ein Desoxynucleosidtriphosphat ist.

9. Verfahren nach einem der Ansprüche 1 bis 5, wobei das Nucleosidtriphosphat, das das Mittel zum Nachweis des Einbaus des Nucleosidtriphosphats in ein Nucleinsäurepolymer enthält, ein Didesoxynucleosidtriphosphat ist.

10. Verfahren nach einem der Ansprüche 1 bis 5, wobei das Gemisch ein zweites Nucleosidtriphosphat enthält, das ein vom ersten Mittel unterschiedliches zweites Mittel umfaßt, zum Nachweis des Einbaus des zweiten Nucleosidtriphosphats in ein Nucleinsäurepolymer.

11. Verfahren nach Anspruch 1, wobei das verlängerte Produkt von Schritt (d) eluiert wird, bevor der Einbau des eingebauten Nucleosidtriphosphats festgestellt wird.

12. Verfahren nach Anspruch 2, wobei die Nucleotidvariationen in einem einzigen Schritt durch Zugabe einer Vielzahl von Nachweisschritt-Primern und unterschiedlich markierten Nucleosidtriphosphaten zur Identifizierung der unterschiedlichen Nucleotidreste nachgewiesen werden.

13. Verfahren nach Anspruch 4, wobei der Mikroorganismus HIV ist.

14. Verfahren nach Anspruch 13, wobei die Punktmutation an einer Stelle auftritt, die ausgewählt ist aus Asp 67, Lys 80 und Thr 215.

15. Verfahren nach Anspruch 5, wobei die Zellen Lymphocyten sind.

16. Verfahren nach Anspruch 15, wobei die Zellen leukämische Zellen sind.

17. Kit zur Verwendung in der Bestimmung spezifischer Nucleotidvariationen in einem Ziel-Nucleinsäurepolymer, umfassend in verpackter Kombination
(a) ein Paar von Amplifikationsoligonucleotidprimern, wobei ein Primer komplementär ist zu und hybridisiert mit einem Teil einer der Stränge des doppelsträngigen Zielnucleinsäurepolymers und der andere komplementär ist zu und hybridisiert mit einem Teil des anderen Stranges der Zielnucleinsäure und auch eine erste Verknüpfungseinheit umfaßt, wobei die erste Verknüpfungseinheit eine Hälfte eines Affinitätspaares ist, ausgewählt aus Biotin-Avidin/Streptavidin, Antigene/Haptene-Antikörper oder Schwermetallderivate-Thiogruppen, und wobei das Oligonucleotidprimerpaar wirksam ist als Primer für enzymatische Nucleinsäurepolymerisation;
(b) mindestens einen Nachweisschritt-Primer, umfassend ein Oligonucleotid, das komplementär ist zu und hybridisiert mit einem Teil, der 3' zu einem variablen Nucleotid des Ziel-Nucleinsäurepolymers liegt; und gegebenenfalls
(c) mindestens einen festen Träger, umfassend eine feste Matrix und mindestens eine Verknüpfungsstelle, die das Oligonucleotid der Amplifikationssonde über die erste Verknüpfungseinheit immobilisieren kann; und
(d) mindestens ein Nucleosidtriphosphat, das Mittel zum Nachweis des Einbaus des Nucleosidtriphosphats in ein Nucleinsäurepolymer enthält.

18. Kit nach Anspruch 17 zur Verwendung bei der Identifizierung der Nucleotidvariation von Apolipoprotein E-Polymorphismus, wobei der Nachweisschritt-Primer die Sequenz
5'-GCG CGG ACA TGG AGG ACG TG umfaßt.

19. Kit nach Anspruch 17 zur Verwendung bei der Identifizierung der Nucleotidvariation von Apolipoprotein E-Polymorphismus, wobei der Nachweisschritt-Primer die Sequenz
5'-ATG CCG ATG ACC TGC AGA AG umfaßt.

20. Kit nach Anspruch 17 zur Verwendung bei der Identifizierung der Nucleotidvariation von Apolipoprotein E-Polymorphismus, wobei der Nachweisschritt-Primer die Sequenz
5'-GTA CTG CAC CAG GCG GCC GC umfaßt.

21. Kit nach Anspruch 17 zur Verwendung bei der Identifizierung der Nucleotidvariation von Apolipoprotein E-Polymorphismus, wobei der Nachweisschritt-Primer die Sequenz
5'- GGC CTG GTA CAC TGC CAG GC umfaßt.

22. Kit nach Anspruch 17 zur Verwendung beim Nachweis der Nucleotidvariation im Codon 6 des menschlichen b-Globingens, das Sichelzellenanämie verursacht, wobei der Nachweisschritt-Primer die Sequenz
5'-CAT GGT GCA CCT GAC TGG TG umfaßt.

23. Kit nach Anspruch 17 zur Verwendung beim Nachweis der Nucleotidvariation im Codon 6 des menschlichen b-Globingens, das Sichelzellenanämie verursacht, wobei der Nachweisschritt-Primer die Sequenz
5'-CAG TAA CGG CAG GCG GCC GC umfaßt.

24. Kit nach Anspruch 17 zur Verwendung beim Nachweis einer Nucleotidvariation im Codon 12 des K-ras-Gens, wobei der Nachweisschritt-Primer die Sequenz
5'-AAG GCA CTC TTG CCT ACG CCA umfaßt.

25. Kit nach Anspruch 17 zur Verwendung beim Nachweis einer Nucleotidvariation im Codon 12 des K-ras-Gens, wobei der Nachweisschritt-Primer die Sequenz
5'-AGG CAC TCT TGC CTA CGC CAC umfaßt.

26. Kit nach Anspruch 17 zur Verwendung beim Nachweis einer Nucleotidvariation im Codon 12 des K-ras-Gens, wobei der Nachweisschritt-Primer die Sequenz
5'-AAC TTG TGG TAG TTG GAG CT umfaßt.

27. Kit nach Anspruch 17 zur Verwendung beim Nachweis einer Nucleotidvariation im Codon 12 des K-ras-Gens, wobei der Nachweisschritt-Primer die Sequenz
5'-ACT TGT GGT AGT TGG AGC TG umfaßt.

28. Kit nach Anspruch 17 zur Verwendung beim Nachweis einer Nucleotidvariation im Codon 12 des N-ras-Gens, wobei der Nachweisschritt-Primer die Sequenz
5'-ACT GGT GGT GGT TGG AGC AG umfaßt.

29. Kit nach Anspruch 17 zur Verwendung beim Nachweis von Resistenz gegenüber AZT in HIV-1-Viren, wobei der Nachweisschritt-Primer die Sequenz
5'-ATC TGT TGA GGT GGG GAC TT umfaßt.

30. Kit nach Anspruch 17 zur Verwendung beim Nachweis von cystischer Fibrose, wobei der Nachweisschritt-Primer die Sequenz 5'-TGG CAC CAT TAA AGA AAA TAT CAT umfaßt.

## Revendications

1. Procédé de détection d'une variation particulière d'un nucléotide en un site défini dans un polynucléotide cible où un premier résidu nucléotidique est remplacé par un second résidu nucléotidique, lequel procédé comprend les étapes consistant
a) à effectuer une réaction d'amplification modifiée dans laquelle une des deux- amorces d'amplification comprend un premier groupement de fixation lié à l'amorce, de manière à obtenir un produit d'amplification double-brin dans lequel un seul des brins comporte un premier groupement de fixation, ledit premier groupement de fixation correspondant à une moitié d'un couple d'affinité choisi dans le groupe formé par les couples biotine-avidine/streptavidine, antigène/haptène ou dérivé d'un métal lourd/groupes thiol ;
b) puis, dans n'importe quel ordre, à séparer le produit d'amplification obtenu dans l'étape a) en simples brins et à immobiliser le brin comprenant le premier groupement de fixation sur un support solide à l'aide de l'autre composant du couple d'affinité, toutes les substances non liées étant ensuite éliminées ;
c) à hybrider une quantité détectable de l'acide nucléique cible simple brin immobilisé obtenu dans l'étape b) avec une amorce de détection comprenant plusieurs résidus nucléotidiques, ladite amorce étant complémentaire à la séquence nucléotidique à laquelle on s'intéresse dans une région située vers l'extrémité 3' par rapport au site défini de sorte que, lorsque l'amorce est hybridée avec l'acide nucléique cible, il n'y ait pas de résidus nucléotidiques entre le site défini et l'extrémité 3' de l'amorce de détection qui soient identiques aux premier ou deuxième résidu nucléotidique à détecter ;
d) à allonger l'amorce à l'aide d'un agent de polymérisation dans un mélange comprenant un ou plusieurs nucléosides-triphosphate, le mélange contenant au moins un nucléoside-triphosphate complémentaire au premier ou au deuxième résidu nucléotidique et qui comprend un moyen permettant de détecter l'incorporation du nucléoside-triphosphate dans un polynucléotide, et éventuellement un ou plusieurs nucléosides-triphosphate de terminaison de chaîne ; et
e) à détecter l'incorporation du nucléoside-triphosphate en utilisant ledit moyen, l'identité du résidu nucléotidique au niveau du site défini étant ainsi révélée.

2. Procédé permettant de détecter une multitude de variations nucléotidiques particulières en des sites définis dans un polynucléotide cible, dans lequel au moins un premier résidu nucléotidique est remplacé par un second résidu- nucléotidique en un premier site défini et un troisième nucléotide est remplacé par un quatrième résidu nucléotidique en un second site défini, la première variation nucléotidique étant détectée par application du procédé selon la revendication 1, et les variations nucléotidiques suivantes étant détectées, après élimination de la première amorce de détection allongée, par application des étapes c), d) et e) du procédé de la revendication 1 au brin déjà immobilisé, pour chacune des autres variations nucléotidiques.

3. Procédé de détection, chez un patient ayant une prédisposition à une abérration génétique résultant d'une variation nucléotidique particulière en un site défini du matériel génétique du patient dans lequel un premier résidu nucléotidique est remplacé par un second résidu nucléotidique, caractérisé en ce que l'on applique le procédé de la revendication 1 pour détecter ladite variation sur un échantillon contenant une quantité détectable de matériel génétique prélevé chez le patient.

4. Procédé de détection d'une mutation ponctuelle en un site défini du génome d'un microorganisme modifiant le caractère pathogène du microorganisme ou lui conférant une résistance à des agents thérapeutiques, dans lequel un premier nucléotide est remplacé par un second résidu nucléotidique, caractérisé en ce que l'on applique le procédé de la revendication 1 pour détecter ladite mutation ponctuelle sur un échantillon contenant une quantité détectable de matériel génétique provenant du microorganisme.

5. Procédé de détection de cellules comportant une mutation ponctuelle en un site défini de leur matériel génétique, où un premier résidu nucléotidique est remplacé par un deuxième résidu nucléotidique, quand lesdites cellules sont mélangées à une population de cellules, caractérisé en ce que l'on applique le procédé de la revendication 1 pour détecter ladite mutation ponctuelle sur une quantité détectable de matériel génétique provenant de la population de cellules dans lequel le rapport cellules mutées/cellules non mutées est conservé.

6. Procédé selon les revendications 1 à 5 dans lequel l'amorce de détection est complémentaire à une région de la séquence nucléotidique à laquelle on s'intéresse s'étendant du résidu nucléotidique immédiatement adjacent au site défini vers l'extrémité 3' du polynucléotide cible.

7. Procédé selon les revendications 1 à 5 dans lequel les amorces de détection sont immobilisées pendant ou après l'étape d'allongement de l'amorce de détection d).

8. Procédé selon les revendications 1 à 5 dans lequel le nucléoside-triphosphate comprenant un moyen de détection de l'incorporation du nucléoside-triphosphate dans un acide nucléique est un désoxynucléoside-triphosphate.

9. Procédé selon les revendications 1 à 5 dans lequel le nucléoside-triphosphate comprenant un moyen de détection de l'incorporation du nucléoside-triphosphate dans un acide nucléique polymère est un didésoxynucléoside-triphosphate.

10. Procédé selon les revendications 1 à 5 dans lequel le mélange contient un second nucléoside-triphosphate comprenant un deuxième moyen, différent dudit premier moyen, pour détecter l'incorporation du second nucléoside-triphosphate dans un acide nucléique polymère.

11. Procédé selon la revendication 1 dans lequel le produit d'allongement obtenu dans l'étape (d) est élué avant la détermination de l'incorporation du nucléoside-triphosphate à incorporer.

12. Procédé selon la revendication 2 dans lequel les variations nucléotidiques sont détectées en une seule étape par addition de plusieurs amorces de détection et de nucléosides-triphosphate marqués de différentes manières permettant l'identification des différents résidus nucléotidiques.

13. Procédé selon la revendication 4 dans lequel le microorganisme est le VIH.

14. Procédé selon la revendication 13 dans lequel la mutation ponctuelle se trouve en un site choisi parmi Asp 67, Lys 80 et Thr 215.

15. Procédé selon la revendication 5 dans lequel les cellules sont des lymphocytes.

16. Procédé selon la revendication 15, dans lequel les cellules sont des cellules leucémiques.

17. Kit destiné à être utilisé pour la détection de variations nucléotidiques spécifiques dans un acide nucléique polymère comprenant, dans un emballage combiné,
(a) une paire d'amorces oligonucléotidiques d'amplification, dans laquelle une des amorces est complémentaire à une région d'un des brins du polynucléotide cible double-brin et capable de s'hybrider avec celle-ci, et l'autre amorce est complémentaire à une région de l'autre brin dudit polynucléotide cible et capable de s'hybrider avec celle-ci et comprend également un premier groupe de fixation, ledit premier groupe de fixation étant une moitié d'un couple d'affinité choisi parmi les couples biotine-avidine/streptavidine, antigène/haptène ou dérivé d'un métal lourd/groupes thiol, laquelle paire d'amorces oligonucléotidiques est efficace en tant qu'amorce pour une polymérisation enzymatique d'acide nucléique ;
(b) au moins une amorce de détection comprenant un oligonucléotide complémentaire à une région située vers l'extrémité 3' d'un nucléotide variable du polynucléotide cible et peut s'hybrider avec cette région, et facultativement
(c) au moins un support solide comprenant une matrice solide et au moins un site de fixation capable d'immobiliser l'oligonucléotide de l'échantillon d'amplification par l'intermédiaire du premier groupement de fixation, et
(d) au moins un nucléoside-triphosphate contenant un moyen permettant de détecter l'incorporation du nucléoside-triphosphate dans un polynucléotide.

18. Kit selon la revendication 17 destiné à être utilisé pour l'identification de la variation nucléotidique caractéristique du polymorphisme de l'apolipoprotéine E, dans lequel l'amorce de détection comprend la séquence 5'-GCG CGC ACA TGG AGG ACG TG.

19. Kit selon la revendication 17 destiné à être utilisé pour l'identification de la variation nucléotidique caractéristique du polymorphisme de l'apolipoprotéine E, dans lequel l'amorce de détection comprend la séquence 5'-ATG CCG ATG ACC TGC AGA AG.

20. Kit selon la revendication 17 destiné à être utilisé pour l'identification de la variation nucléotidique caractéristique du polymorphisme de l'apolipoprotéine E, dans lequel l'amorce de détection comprend la séquence 5'-GTA CTG CAC CAG GCG GCC GC.

21. Kit selon la revendication 17 destiné à être utilisé pour l'identification de la variation nucléotidique caractéristique du polymorphisme de l'apolipoprotéine E, dans lequel l'amorce de détection comprend la séquence 5'-GGC CTG GTA CAC TGC CAG GC.

22. Kit selon la revendication 17 destiné à être utilisé pour la détection de la variation nucléotidique du codon 6 du gène codant la β-globuline humaine responsable de la drépanocytose, dans lequel l'amorce de détection comprend la séquence 5'-CAT GGT GCA CCT GAC TGG TG.

23. Kit selon la revendication 17 destiné à être utilisé pour la détection de la variation nucléotidique du codon 6 du gène codant la β-globuline humaine responsable de la drépanocytose, dans lequel l'amorce de détection comprend la séquence 5'-CAG TAA CGG CAG GCG GCC GC.

24. Kit selon la revendication 17 destiné à être utilisé pour la détection de la variation nucléotidique du codon 12 du gène K-ras, dans lequel l'amorce de détection comprend la séquence 5'-AAG GCA CTC TTG CCT ACG CCA.

25. Kit selon la revendication 17 destiné à être utilisé pour la détection de la variation nucléotidique du codon 12 du gène K-ras, dans lequel l'amorce de détection comprend la séquence 5'-AGG CAC TCT TGC CTA CGC CAC.

26. Kit selon la revendication 17 destiné à être utilisé pour la détection de la variation nucléotidique du codon 12 du gène K-ras, dans lequel l'amorce de détection comprend la séquence 5'-AAC TTG TGG TAG TTG GAG CT.

27. Kit selon la revendication 17 destiné à être utilisé pour la détection de la variation nucléotidique du codon 12 du gène K-ras, dans lequel l'amorce de détection comprend la séquence 5'-ACT TGT GGT AGT TGG AGC TG.

28. Kit selon la revendication 17 destiné à être utilisé pour la détection de la variation nucléotidique du codon 12 du gène N-ras, dans lequel l'amorce de détection comprend la séquence 5'-ACT GGT GGT GGT TGG AGC AG.

29. Kit selon la revendication 17 destiné à être utilisé pour la détection de la résistance du virus VIH-1 à l'AZT dans lequel l'amorce de détection comprend la séquence 5'-ATC TGT TGA GGT GGG GAC TT.

30. Kit selon la revendication 17 destiné à être utilisé pour la détection de la fibrose cystique dans lequel l'amorce de détection comprend la séquence 5'-TGG CAC CAT TAA AGA AAA TAT CAT.
